(19) Europäisches Patentamt
European Patent Office
Office européen des brevets

(11) EP 0 677 042 B1

(12) # FASCICULE DE BREVET EUROPEEN

(45) Date de publication et mention de la délivrance du brevet:
**22.07.1998 Bulletin 1998/30**

(21) Numéro de dépôt: **94903929.1**

(22) Date de dépôt: **29.12.1993**

(51) Int Cl.[6]: **C07D 209/16**, A61K 31/40, C07D 409/12

(86) Numéro de dépôt international:
**PCT/FR93/01317**

(87) Numéro de publication internationale:
**WO 94/15916 (21.07.1994 Gazette 1994/17)**

(54) **LIGANDS SELECTIFS DES RECEPTEURS 5HT1D-5HT1B DERIVES D'INDOLE-PIPERAZINE UTILES COMME MEDICAMENTS**

INDOL-PIPERAZIN-DERIVATE ALS SELEKTIVE LIGANDEN DER 5HT1D UND 5HT1B RECEPTOREN VERWENDBAR ALS ARZNEIMITTEL

SELECTIVE LIGANDS OF 5HT1D-5HT1B RECEPTORS DERIVED FROM INDOLE-PIPERAZINE USEFUL AS MEDICAMENTS

(84) Etats contractants désignés:
**AT BE CH DE DK ES FR GB GR IE IT LI LU MC NL PT SE**

(30) Priorité: **30.12.1992 FR 9215919**

(43) Date de publication de la demande:
**18.10.1995 Bulletin 1995/42**

(73) Titulaire: **PIERRE FABRE MEDICAMENT**
**92100 Boulogne (FR)**

(72) Inventeurs:
- **HALAZY, Serge**
  **F-81090 Lagarrigue (FR)**
- **PEREZ, Michel**
  **F-81100 Castres (FR)**
- **BRILEY, Michael**
  **F-81100 Castres (FR)**

(74) Mandataire: **Ahner, Francis et al**
**CABINET REGIMBEAU**
**26, avenue Kléber**
**75116 Paris (FR)**

(56) Documents cités:
**EP-A- 0 457 701** **GB-A- 2 082 175**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 677 042 B1

Printed by Jouve, 75001 PARIS (FR)

## Description

La présente invention se rapporte à de nouveaux composés indoliques dérivés de pipérazine, à des procédés pour leur préparation et à leurs utilisations thérapeutiques.

Au cours des vingt dernières années, des progrès considérables ont été accomplis dans la compréhension de la biochimie et de la physiologie de la sérotonine ou 5-hydroxytryptamine (5-HT) tant au niveau du système nerveux central qu'au niveau cardiovasculaire. C'est ainsi qu'il a été démontré que la sérotonine pouvait jouer un rôle dans certaines maladies telles que la dépression, la douleur, les désordres compulsifs obsessionnels, l'obésité, la schizophrénie, l'anxiété, certains dysfonctionnements sexuels, la migraine et autres désordres vasospasmodiques. La découverte des différentes sous-classes des récepteurs de la sérotonine a stimulé la recherche de ligands sélectifs (cf. R. A. Glennon, Neuroscience & Biobehavioral Reviews, 14, 35-47, 1990 ; A.W. Schmidt, S.J. Peroutka, FASEB J. 3, 2242-2249, 1989) afin de mieux cerner la signification pharmacologique de chacun de ces sous-types de récepteurs, et de pouvoir identifier de nouveaux agents thérapeutiques sélectifs, non toxiques et dénués d'effets secondaires indésirables (S. Langer, N. Brunello, G. Racagni, J. Mendlewicz, "Serotonin receptors subtypes : pharmacological significance and clinical implications" Karger ed. (1992) ; B.E. Leonard, Int. Clin. Psychopharmacology, 7, 13-21 (1992) ; D.G. Grahame-Smith, Int. Clin. Psychopharmacology, 6, suppl. 4, 6-13 (1992)).

Les composés selon la présente invention sont des ligands puissants et sélectifs des récepteurs de la 5-hydroxytryptamine et plus particulièrement du récepteur récemment décrit comme récepteur $5HT_{1B}$ et/ou $5HT_{1D}$. Les médicaments selon la présente invention trouvent leur emploi dans le traitement tant curatif que préventif des désordres liés à la sérotonine.

La demande de brevet d'invention FR267 1971 décrit une classe de dérivés 5-O-carboxyméthylés de la tryptamine qui sont doués d'une bonne affinité pour les récepteurs $5HT_{1D}$ et peuvent en conséquence agir comme agents thérapeutiques pour le traitement de la migraine. Néanmoins, l'application FR 2 671 971 en aucun cas, ne décrit ni ne suggère les dérivés de pipérazine décrits dans la présente invention.

La présente invention concerne des composés de formule :

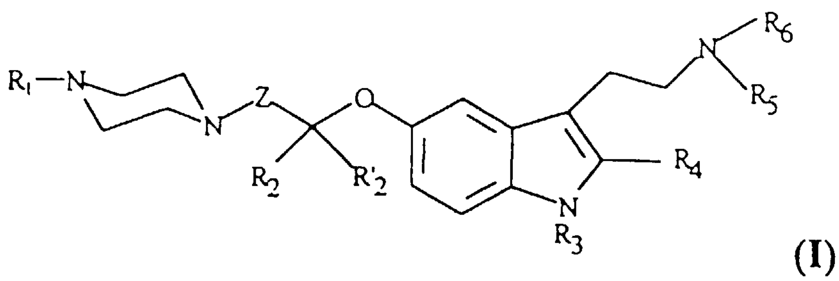

(I)

leur préparation et les médicaments les contenant.

Dans la formule (I) :

$R_1$ représente un atome d'hydrogène, un radical alkyle ramifié ou linéaire en $C_1$ à $C_6$ ou un radical phényle, benzyle, cycloalkyle en $C_3$ à $C_7$, polycycloalkyle en $C_7$ à $C_{12}$, dibenzocycloalkyle, dibenzooxépine, dibenzoazépine, dibenzothiépine, benzopyrrolocycloaikyle, benzothiénocycloalkyle, naphtyle, éventuellement substitués par un ou plusieurs substituants choisis parmi les atome d'halogène et les radicaux alkyle en $C_1$ à $C_6$, alcoxy et alkylthio dont les fragments alkyle sont en $C_1$ à $C_6$,

Z représente C=O, $SO_2$, ou $(CH_2)_n$ dans lequel n est compris entre 1 et 5,

$R_2$ représente un atome d'hydrogène, un radical alkyle ramifié ou linéaire en $C_1$ à $C_6$, ou, un radical phényle, benzyle, cycloalkyle, pyrrole, furane, pyridinyle, thiophényle, éventuellement substitués par un ou plusieurs substituants choisis parmi les atomes d'halogène et les radicaux alkyle, alcoxy et alkylthio dont les fragments alkyle sont en $C_1$ à $C_6$,

$R'_2$ représente un atome d'hydrogène, un radical alkyle ramifié ou linéaire en $C_1$ à $C_6$ ou un radical phényle.

$R_3$ représente un atome d'hydrogène, un radical alkyle ramifié ou linéaire en $C_1$ à $C_6$, ou un radical benzyle ou phénéthyle,

$R_4$ représente un atome d'hydrogène, de chlore, de fluor ou de brome ou un radical alkyle linéaire ou ramifié en $C_1$ à $C_6$,

$R_5$ représente un atome d'hydrogène, un radical alkyle ramifié ou linéaire en $C_1$ à $C_6$ ou un radical benzyle ou phénéthyle,

$R_6$ représente un atome d'hydrogène, un radical alkyle linéaire ou ramifié en $C_1$ à $C_6$, un radical acycle ($COR_7$), acyloxy ($CO_2R_7$) ou acylamino ($CONHR_7$) dans lesquels $R_7$ représente un radical alkyle linéaire ou ramifié en $C_1$ à $C_6$, ou un radical phényle,

Dans les définitions qui précèdent et celles qui seront citées ci-après, sauf mention contraire, les radicaux alkyles, alcoxy ou alkylthio contiennent 1 à 6 atomes de carbone en chaîne droite ou ramifiée, les portions cycloalkyle contiennent 3 à 7 atomes de carbone et les portions polycycloalkyle contiennent 7 à 12 atomes de carbone. Dans la formule (I), les atomes d'halogène sont préférentiellement les atomes de chlore, de fluor et de brome.

Les composés de formule (I) contenant 1 ou plusieurs centres asymétriques présentent des formes isomères. Les racémiques et les énantiomères purs de ces composés font également partie de cette invention.

L'invention comprend également les sels et solvats (par exemple hydrates) de ces composés acceptables pour l'usage thérapeutique.

Parmi les sels acceptables pour l'usage thérapeutique des indoles de formule générale (I), on citera des sels formés par addition avec des acides organiques ou minéraux et par exemple les chlorhydrates, les bromhydrates, les sulfates, les fumarates et les maléates. D'autres sels peuvent être utiles dans la préparation des composés de formule (I), par exemple les adduits avec le sulfate de créatinine.

Les composés de formule générale (I) peuvent aussi être utilisés sous la forme de bioprécurseurs dont la structure diffère de celle des composés de formule (I), mais qui, administrés à un animal ou à un être humain, sont convertis dans l'organisme en un composé de formule (I).

Une classe appréciée de composés selon l'invention consiste en ceux qui répondent à la formule générale (Ia) :

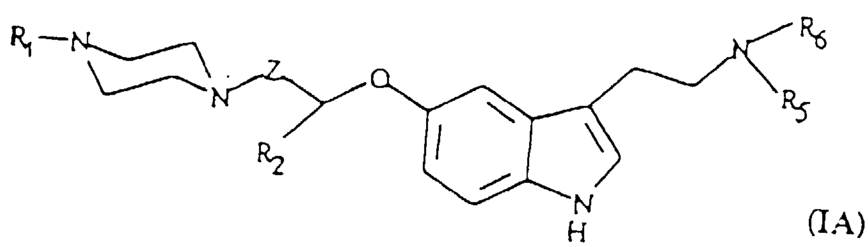

(IA)

dans laquelle $R_1$, $R_2$, $R_5$ et $R_6$ sont définis comme précédemment dans la formule (I) et leurs sels et solvats (par exemple hydrates) acceptables pour l'usage thérapeutique.

L'invention comprend également la préparation par les procédés généraux décrits ci-après des composés de formule générale (I) et de leurs sels et solvats (par exemple hydrates) acceptables pour l'usage thérapeutique.

Selon un premier procédé général (A) on peut préparer un composé de formule générale (I) en faisant réagir un composé de formule générale (II) :

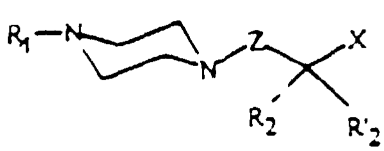

(II)

dans laquelle $R_1$, Z, $R_2$, $R'_2$ sont définis comme dans la formule (I) et X est défini comme un groupe partant tel qu'un halogène (de préférence un atome de brome, d'iode ou de chlore) un mésylate, un tosylate ou un triflate avec un dérivé de la sérotonine de formule générale (III) :

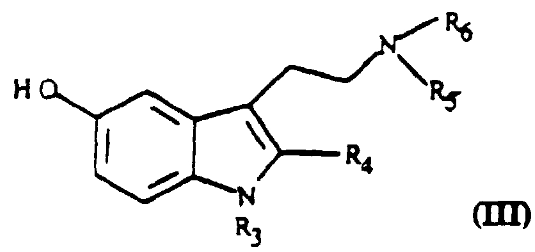

(III)

dans laquelle les résidus $R_3$, $R_4$, $R_5$ et $R_6$ sont définis comme décrits précédemment dans la formule (I) si ce n'est que $R_6$ ne peut être un hydrogène. La préparation des dérivés de formule (I) dans lesquels $R_6$ est un hydrogène est effectuée par hydrolyse d'un dérivé de formule (I) dans lequel $R_6$ est un groupe COR ou $CO_2R_7$, de préférence $CO_2R_7$ dans lequel $R_7$ est préférentiellement un résidu $^t$butyle ou benzyle. La transformation des composés de formule (I) dans lesquels $R_6$ est un groupe $CO_2{}^tBu$ (BOC) en composés de formule (I) dans lequels $R_6$ est un hydrogène est préférentiellement effectuée à l'aide d'un acide (tel que l'acide chlorhydrique ou l'acide trifluoroacétique) dans un solvant organique tel que l'éther, le tétrahydrofurane, le toluène, le dichlorométhane ou le chloroforme à une température comprise entre -15°C et 40°C.

La transformation des composés de formule (I) dans lesquels $R_6$ est un groupe $CO_2CH_2C_6H_5$ (communément appelé Z) en composés de formule (I) dans lesquels $R_6$ est un hydrogène est préférentiellement effectuée par hydrogénation catalytique en utilisant préférentiellement du palladium sur charbon comme catalyseur, sous pression atmosphérique d'hydrogène, dans un solvant tel que le THF, l'éthanol, l'isopropanol pouvant contenir jusqu'à 10 % d'acide acétique ou citrique, à une température comprise entre 0° et 40°C.

La préparation des dérivés de formule (I) par condensation des dérivés de formule (II) avec les dérivés de formule (III) peut être réalisée, d'une manière générale, en présence d'une base organique (NaH, KH, $Et_3N$, DBU, DBN, TMP, DIPEA, $^t$BuOK) ou inorganique ($K_2CO_3$, $KHCO_3$, $NaHCO_3$, $Cs_2CO_3$, KOH, NaOH, $CaCO_3$...) dans un solvant anhydre tel que le THF, la DMF, le DMSO, l'acétone, la diéthylcétone, la méthyléthylcétone, l'acétonitrile ou la DME à une température comprise entre 20° et 140°C, en présence ou non d'un sel comme catalyseur et qui peut être KI, $Bu_4NI$, LiI, $AgBF_4$, $AgClO_4$, $Ag_2CO_3$, KF, $Bu_4NF$ ou CsF. Le choix des conditions expérimentales pour réaliser la condensation entre les dérivés de formule (II) et (III) pour obtenir les dérivés de formule (I) est bien évidemment dépendant de la nature des substituants dans les réactifs (II) et (III) et plus particulièrement de la nature des groupements Z, $R_2$ et $R'_2$. A titre d'exemple, lorsque Z est une fonction carbonyle (CO), $R_2$ est un atome d'hydrogène et X un halogène, la condensation entre (II) et (III) pour donner (I) est effectuée préférentiellement à 80°C, dans la méthyléthylcétone, en présence d'un excès de $K_2CO_3$ et d'une quantité catalytique de KI. Lorsque Z est un groupe carbonyle et que $R_2$ et $R'_2$ sont tous deux différents d'un hydrogène, la méthode préférée consiste à faire réagir un dérivé de formule (III) avec ce dérivé de formule (II), en présence d'un sel d'argent tel que le tétrafluoroborate d'argent et d'une base inorganique telle que $K_2CO_3$. Lorsque le groupe Z est défini comme $(CH_2)_n$ la condensation entre les dérivés (II) et (III) est effectuée dans un solvant tel que la DMF ou le DMSO, en présence d'une base telle que la DBU ou la DIPEA, à 100°C en présence de KI ou de $Bu_4NI$ en quantité catalytique. Une méthode alternative et particulièrement appréciée consiste à condenser les dérivés (II) et (III), en condition neutre, dans la DMF, en présence d'un large excès d'un fluorure tel que KF, CsF ou $Bu_4NF$.

Les composés de formule générale (II) dans laquelle les substituants $R_1$, $R_2$, $R'_2$ et X sont définis comme précédemment sont préparés par des méthodes qui diffèrent en fonction de la nature du résidu Z. C'est ainsi que les dérivés de formule (II) dans lesquels Z est un groupe carbonyle faisant parti d'une fonction amide sont obtenus par réaction de dérivés de pipérazine de formule générale (IV)

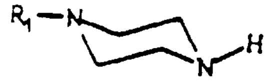

(IV)

dans laquelle le résidu $R_1$ est défini comme dans la formule (I), avec un dérivé de formule (V)

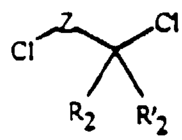

(V)

dans laquelle $R_2$ et $R'_2$ sont définis comme dans la formule (I) et Z représente C=O. Cette réaction qui permet de préparer les dérivés de formule (II) dans lesquels Z=CO et X=Cl à partir des dérivés de pipérazines (IV) et des chlorures d'acide (V) est une réaction bien connue de formation d'amide à partir d'une amine et d'un chlorure d'acide et peut être réalisée dans un solvant tel que le dichlorométhane, le THF, le chloroforme, l'acétone, la méthyléthylcétone, la DME ou l'acétonitrile, à une température comprise entre -20°C et 80°C, en présence d'une base telle qu'une amine tertiaire (DBU, $Et_3N$, DIPEA) ou des bases inorganiques telles que des carbonates ($KHCO_3$, $NaHCO_3$, $K_2CO_2$, $Na_2CO_3$, $CaCO_3$, $Cs_2CO_3$) la soude ou encore la potasse.

Les dérivés de formule (II) dans lesquels Z représente un groupe $-(CH_2)_n-$ sont généralement préparés par condensation d'un dérivé de pipérazine de formule (IV) avec un dérivé de formule (VI)

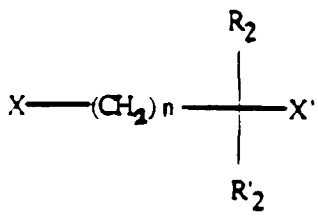

(VI)

dans laquelle X représente un groupe partant tel qu'un chlore, un brome, un iode, un groupe mésylate, tosylate, triflate, $R_2$ et $R'_2$ sont définis comme dans la formule (I) et X' peut être soit identique à X, soit représente un groupe OR' dans lequel R' est défini comme un groupe protecteur d'un alcool tel qu'un éther silylé ($SiMe_3$, $Si^tBuMe_2$, $SiC_6H_5Me_2$), un tétrahydropyrane ou encore un benzyle ou un trityle. Il est bien entendu que, dans le cas où X' est différent de X, la condensation entre le dérivé de pipérazine (IV) et l'intermédiaire (VI) est suivi de l'hydrolyse du groupe protecteur OR' pour donner un dérivé alcoolique intermédiaire qui est transformé en groupe partant ce qui conduit aux composés (II) dans lesquels $R_1$, $R_2$, $R'_2$ et X sont définis comme précédemment. Dans la procédure mentionnée ci-dessus, l'hydrolyse de la fonction OR' en alcool est réalisée par les méthodes décrites et appropriées en fonction de la nature de R' (se référer à l'ouvrage de T.W. Greene, "Protective groups in organic synthesis", John Wiley & Sons, 1981) et la transformation de l'alcool ainsi obtenu en groupe partant (de façon à obtenir les composés (II)) est réalisée par les techniques et méthodes bien connues pour ce type de transformation, telles que l'utilisation de $SOCl_2$ ou $POCl_3$ dans le dichlorométhane pour la formation de dérivés de formule (II) dans lesquels X=Cl, l'utilisation de $PBr_3$ ou $Br_2PO_3$ pour la formation de dérivés de formule (II) dans lesquels X=Br, l'utilisation de $PI_3$ ou $P_2I_4$ pour la formation de dérivés de formule (II) dans lesquels X=I, l'utilisation du chlorure de tosyle pour la formation de dérivés de formule (II) dans lesquels X=Tos, l'utilisation du chlorure de mésyle pour la formation de dérivés de formule (II) dans lesquels X = Mes et enfin l'utilisation d'anhydride triflique pour la formation de dérivés de formule (II) dans lesquels X=Tf. Dans le cas particulier du composé (II) dans lequel Z=$CH_2$, $R_2$ et $R'_2$ sont des hydrogènes, une méthode de préparation préférée consiste à traiter la pipérazine de formule (IV) avec l'époxyde d'éthylène en présence d'une base telle qu'une amine tertiaire, le $^tBuOK$. LiH, NaH, la soude, $K_2CO_3$ ou $Li_2CO_3$ pour conduire au dérivé de formule (II) dans laquelle Z=$CH_2$, $R_2$ et $R'_2$ sont des hydrogènes, après transformation de l'alcool intermédiaire en groupe partant X comme défini dans la formule (II), après transformation comme décrit précédemment.

Les dérivés de formule (II) dans lesquels Z=$SO_2$ sont généralement préparés par réaction des dérivés de pipérazine de formule générale (IV) dans laquelle le groupe $R_1$ est défini comme dans la formule générale (I) avec un dérivé de formule (V) dans laquelle $R_2$ et $R'_2$ sont définis comme dans la formule (I) et Z représente $SO_2$.

Les composés de formule générale (I) peuvent également être préparés selon un second procédé général (B), en faisant réagir un composé de formule générale (IV) :

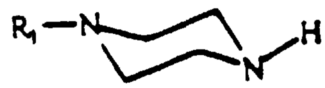

(IV)

dans laquelle $R_1$ est défini comme dans la formule générale (I), avec un dérivé de la sérotonine de formule générale (VII)

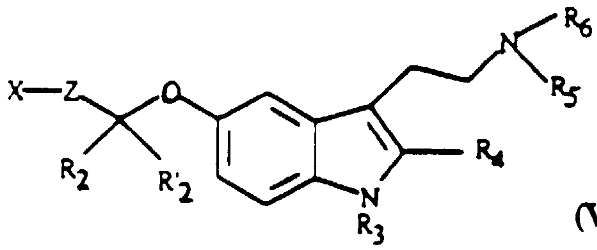

(VII)

dans laquelle les résidus $R_2$, $R'_2$, $R_3$, $R_4$, $R_5$ et $R_6$ sont définis comme décrits précédemment dans la formule si ce n'est que $R_6$ ne peut être un hydrogène et X est défini comme un groupe partant tel qu'un halogène (de préférence un atome de brome, d'iode ou de chlore), un mésylate, un tosylate ou un triflate ou le précurseur d'un groupe partant tel qu'un radical hydroxyle.

La préparation des dérivés de formule (I) dans lesquels $R_6$ est un hydrogène par le procédé général (B) (condensation des intermédiaires (IV) et (VII)) est effectuée par hydrolyse d'un dérivé de formule (I) dans lequel $R_6$ est un groupe protecteur d'amine comme décrit précédemment.

La préparation des dérivés de formule (I) par condensation des intermédaires de formule (IV) avec les dérivés de formule (VII) est réalisée en utilisant des méthodes qui sont liées à la nature du substituant Z. C'est ainsi que la préparation des composés de formule (I) dans lesquels Z est un résidu carbonylé (CO) par le procédé général (B) est réalisée par condensation du dérivé de pipérazine (IV) avec un dérivé de l'acide carboxylique (VII) (X = OH, Z = CO) par les techniques et méthodes bien connues de la synthèse peptidique. C'est ainsi que l'acide carboxylique (VII) (X=OH, Z=CO) peut être préalablement transformé en chlorure d'acide (X=Cl, Z=CO) par réaction avec le chlorure de thionyle, le chlorure d'oxallyle, l'oxychlorure de phosphore, dans un solvant inerte tel que le dichlorométhane ou le chloroforme, l'acétonitrile ou le THF, à une température comprise entre -25°C et +25°C, en présence d'une base aminée telle que la triéthylamine, la DIPEA ou la N-méthyl morpholine. La condensation entre ce chlorure d'acide ainsi obtenu et le dérivé de pipérazine (IV) est alors effectuée dans le même solvant, à une température comprise entre 0 et 50°C pour donner les produits de formule (I) dans lesquels Z représente CO. Une méthode appréciée pour préparer les dérivés de formule (I) dans lesquels Z représente CO par le procédé général (B) consiste à traiter un dérivé d'acide carboxylique de formule (VII) dans lequel X=OH et Z=CO par le chloroformiate d'éthyle dans un solvant tel que le dichlorométhane, le dichloroéthane, le chloroforme ou l'acétonitrile, en présence d'une amine tertiaire telle que la triéthylamine, la diisopropyléthylamine ou la N-méthyl morpholine, à une température comprise entre -20°C et 0°C ; le dérivé de pipérazine est ensuite ajouté au mélange réactionnel qui est agité à une température comprise entre 0° et 35°C pendant un temps allant de 2 à 8 heures.

Une méthode préférée pour préparer les dérivés de formule (I) dans lesquels Z représente CO par le procédé général (B) consiste à traiter l'iodure de 1-méthyl-2-chloro pyridinium par un mélange contenant un acide carboxylique de formule (VII) (dans lequel X=OH et Z=CO), un dérivé de pipérazine de formule (IV), une amine tertiaire telle que la tri-butylamine dans un solvant inerte et anhydre tel que le dichlorométhane, le chloroforme ou l'acétonitrile à une température comprise entre 30°C et 80°C.

La préparation des dérivés de formule (I) dans lesquels Z représente un résidu $-(CH_2)_n-$ par le procédé général (B) est réalisée par condensation entre un dérivé de pipérazine de formule (IV) dans laquelle $R_1$ est défini comme précédemment et un intermédiaire de formule générale (VII) dans lequel X est un groupe partant tel qu'un halogène (préférentiellement un atome de brome, de chlore ou d'iode), un mésylate, un tosylate ou un triflate, Z représente $-(CH_2)-_n$ , $R_2$, $R'_2$, $R_3$, $R_4$, $R_5$ et $R_6$ sont définis comme dans la formule (I). Cette réaction peut être effectuée en présence d'une base organique (NaH, $^tBuOK$, DBU, DIPEA) ou inorganique (KOH, $K_2CO_3$, $NaHCO_3$, $Cs_2CO_3$) dans un solvant anhydre tel que le THF, la DMF, le DMSO, l'acétonitrile ou la méthyléthylcétone à une température comprise entre 20 et 100°C.

Les intermédiaires de formule (VII) peuvent être préparés par condensation d'un dérivé de la sérotonine de formule

(VIII)

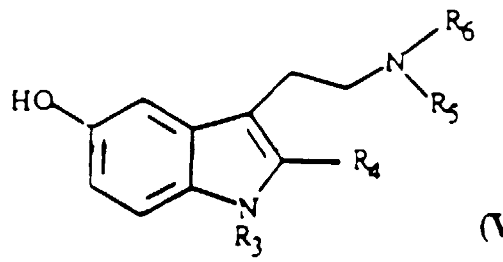

(VIII)

dans laquelle $R_3$, $R_4$, $R_5$ et $R_6$ sont définis comme dans la formule (I) si ce n'est que $R_6$ doit être différent d'un hydrogène avec un dérivé de formule (IX)

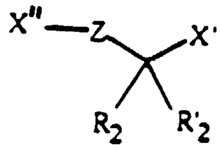

(IX)

dans laquelle X" est un groupe OR où R est un groupement protecteur classique tel que $CH_3$, $C_2H_5$, $CH_2C_6H_5$, ${}^tC_4H_9$ lorsque Z représente $SO_2$ ou CO (il s'agit alors d'esters dans lesquels X"-Z représentent ROCO ou $ROSO_2$) et un groupement silylé (triméthylsilyle, théthylsilyle, tbutyldiméthylsilyle), benzyle, tétrahydropyranyl ou trityl dans le cas où Z représente $-(CH_2)_n-$ et X' représente un groupe partant tel qu'un halogène (de préférence un chlore, un iode ou un brome), un mésylate, un tosylate ou un triflate. Cette réaction de condensation entre les intermédiaires (VIII) et (IX) tels que décrits précédemment est effectuée en milieu basique (en présence d'une base telle que NaH, KH, tBuOK, $K_2CO_3$, $Cs_2CO_3$, DIPEA, DBU) dans un solvant anhydre tel que le DMSO, la DMF, le THF, l'acétonitrile, la méthyléthylcétone ou la DME à une température comprise entre 0°C et 100°C dépendant de la nature de Z. Dans le cas particulier où Z représente CO et où $R_2$, $R'_2$, $R_3$, $R_4$, $R_5$ représentent un hydrogène et $R_6$ un résidu ${}^t$butoxycarbonyle cette réaction a été décrite dans la demande de brevet FR 2671971.

Dans le cas particulier des dérivés de formule (I) dans lesquels $R_1$, $R_3$, $R_4$, $R_5$ sont décrits comme précédemment mais où Z représente $-(CH_2)_n-$ et $R_6$ est différent de COR, une méthode de synthèse préférée consiste à réduire les dérivés correspondants de formule (I) dans lesquels Z représente CO par un agent de réduction qui permet de transformer une amide en amine tel que le borane ($BH_3.Me_2S$) ou $LiAlH_4$ en utilisant les méthodes et techniques bien connues pour ce type de réduction.

Dans le cas particulier des composés de formule (I) dans lesquels $R_1$, $R_3$, $R_4$, $R_5$ et $R_6$ sont décrits comme précédemment, Z représente $SO_2$ et $R_2$, $R'_2$ sont des atomes d'hydrogène, la méthode de préparation plus particulièrement appréciée selon la procédure (B) consiste à condenser un dénvé de la sérotonine de structure (VIII) dans laquelle $R_3$, $R_4$, $R_5$ sont décrits comme précédemment et $R_6$ est différent d'un hydrogène, avec un intermédiaire de formule (IX) dans lequel X" représente OH, Z représente $SO_2$, $R_2$ et $R'_2$ représentent un hydrogène et X' représente un chlore, selon la méthode décrite par H.J. Barber et collaborateurs [J. Appl. Chem. (London), 3, 253 (1953)]. Les produits (VII) ainsi obtenus dans lesquels X" représente OH, $R_2$ et $R'_2$ représentent un hydrogène et Z représente $SO_2$ sont transformés en intermédiaires (VII) dans lesquels X représente un chlore ou un brome et condensés, comme décrits précédemment avec une pipérazine de structure (IV) pour donner le produit de formule (I).

Il faut également considérer comme partie de cette invention la possibilité de transformer des dérivés de formule (I) initialement préparés par les procédés (A) et (B) décrits précédemment en nouveaux dérivés de formule (I), par les techniques et méthodes bien connues de l'homme de métier. C'est ainsi, et à titre d'exemple, que les dérivés de formule (I) dans lesquels $R_3$ représente un hydrogène peuvent être élaborés en dérivés de formule (I) dans lesquels $R_3$ représente un résidu alkyle, benzyle ou acyle par réaction respectivement avec un halogénure d'alkyle, de benzyle ou un chlorure d'acide ou anhydride d'acide, en milieu basique par les méthodes et techniques bien connues pour ce type de réaction et qui, à titre d'exemple, sont décrites dans "The Chemistry of Indoles" édité par R.S. Sundberg volume

18 de "Organic Chemistry, A Series of Monograph", Academic Press, NY, 1970.

On comprendra que dans certaines des transformations ci-dessus, il peut être nécessaire ou souhaitable de protéger des groupes sensibles éventuels de la molécule en question afin d'éviter des réactions secondaires indésirables. Ceci peut être réalisé par l'utilisation des groupes protecteurs conventionnels tels que ceux décrits dans "Protective Groups in Organic Synthesis" ed. J.F. Mc Owie, Plenum Press, 1973 et dans T.W. Greene, Protective Groups in Organic Synthesis, John Wiley & Sons, 1981. Les groupes protecteurs peuvent être enlevés lors de toute étape ultérieure appropriée, en utilisant les méthodes et techniques également décrites dans les références citées précédemment. C'est ainsi que dans certains cas particuliers il peut être nécessaire de protéger l'azote indolique lors de la préparation de composés de formule (I) dans lesquels $R_3$ représente un hydrogène.

Lorsque l'on désire isoler un composé selon l'invention à l'état de sel, par exemple à l'état de sel formé par addition avec un acide, on peut y parvenir en traitant la base libre de formule générale (I) par un acide approprié de préférence en quantité équivalente, ou par le sulfate de créatinine dans un solvant approprié.

Lorsque les procédés décrits ci-dessus pour préparer les composés de l'invention donnent des mélanges de stéréoisomères, ces isomères peuvent être séparés par des méthodes conventionnelles telles que la chromatographie préparative.

Lorsque les nouveaux composés de formule générale (I) possèdent un ou plusieurs centres asymétriques, ils peuvent être préparés sous forme de mélange racémique ou sous forme d'énantiomères que ce soit par synthèse énantiosélective ou par résolution. Les composés de formule (I) possédant au moins un centre asymétrique peuvent par exemple être séparés en leurs énantiomères par les techniques habituelles telles que la formation de paires diastéréomériques par formation d'un sel avec un acide optiquement actif tel que l'acide (-) di-p-toluoyl-1-tartrique, l'acide (+) di-p-toluoyl-1-tartrique. l'acide (+) camphor sulfonique, l'acide (-) camphor sulfonique, l'acide (+) phénylpropionique, l'acide (-)-phénylpropionique, suivie par cristallisation fractionnée et régénération de la base libre. Les composés de formule (I) dans lesquels $R_6$ est un hydrogène comprenant au moins un centre asymétrique peuvent également être résolus par formation d'amides diastéréomériques qui sont séparés par chromatographie et hydrolysés pour libérer l'auxiliaire chiral.

D'une façon générale, les composés de formule (I) peuvent être purifiés par les méthodes habituelles par exemple par cristallisation (en particulier lorsque les composés de formule (I) sont isolés sous forme de sel), chromatographie ou extraction.

Les exemples qui suivent illustrent l'invention sans toutefois en limiter la portée.

**Exemple 1 - Chlorhydrate de la 4-*o*-tolyl 1-(tryptamine 5-O-carboxyméthyl) pipérazide :**

METHODE B

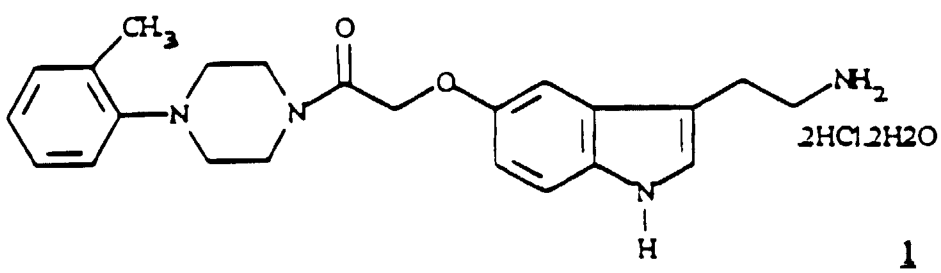

1

**1A - terbutoxycarbamate de la 5-hydroxytryptamine :**

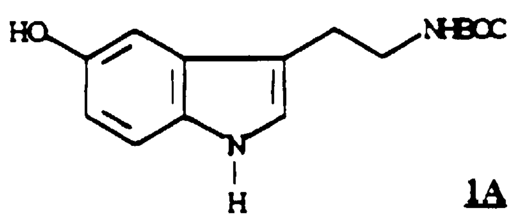

1A

Le sel créatine sulfate monohydrate de la sérotonine (20 g, 49,3 mmol) est traité par le diterbutyle dicarbonate (16,1 g, 74 mmol) dans l'eau (360 ml) en présence de soude 2N (72 ml) à température ambiante. Après 1 heure la réaction est diluée par de l'acétate d'éthyle (600 ml) et agitée pendant 10 minutes. Les 2 phases formées sont séparées par décantation; la phase organique est lavée à l'eau, séchée sur sulfate de sodium, filtrée puis évaporée à sec. Le sirop obtenu est chromatographie sur colonne de gel de silice éluée par un mélange dichlorométhane/méthanol (20: 1 ; v/v). Le composé pur est isolé sous forme de sirop marron (11,1 g ; 81 %).
Analyse élémentaire ($C_{15}H_{20}N_2O_3$), % calculés : C 65,20 : H 7,30 : N 10,14; % trouvés : C 64.15 ; H 7.47 : N 9.77.
Spectre de résonance magnétique nucléaire du proton, $CDCl_3$ (ppm) : 1,44 s, 9H (tBu) ; 2,86-t, 2H ($CH_2$) ; 3,45 m, 2H ($CH_2$) ; 4,68 s, 1H (NH) ; 5,59 s, 1H (O-H) ; 6,77-7,26 m, 4H (Ar) ; 7,99 s, 1H (NH).

**1B - terbutoxycarbamate de la tryptamine 5-(méthyl O-acétate) :**

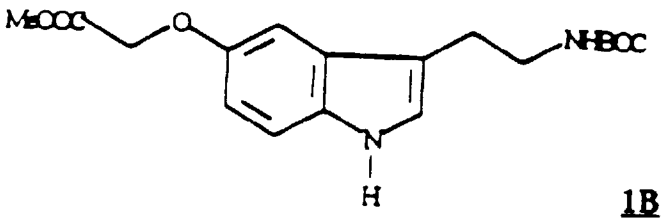

**1B**

Le composé 1A (5,5 g ; 20,07 mmol) en solution dans la méthyléthylcétone (70ml) en présence de carbonate de potassium (6,9 g ; 50,1 mmol) et d'ioduré de potassium (33 mg ; 0,2 mmol) est traité, goutte à goutte, par le bromoacétate de méthyle (3,3 ml; 36,1 mmol). Le mélange est ensuite porté à reflux pendant 5 heures, ramené à température ambiante, filtré sur célite et évaporé à sec. Le sirop est repris dans l'éther éthylique, lavé à la soude 0,5N puis à l'eau. La phase organique est séchée sur sulfate de sodium, filtrée puis évaporée à sec. Le solide jaune obtenu est chromatographié sur colonne de gel de silice éluée par un mélange chloroforme/méthanol/ammoniaque (95:4,5:0,5 ; v/v). Le composé pur est isolé (6,4 g ; 91 %).
Analyse élémentaire ($C_{18}H_{24}N_2O_5$), % calculés: C 62,06 ; H 6,94 ; N 8,04 ; % trouvés : C 61,44; H 6,88 ; N 7,52.
Spectre de résonance magnétique nucléaire du proton, $CDCl_3$ (ppm) : 1,44 s, 9H (tBu) ; 2,88 t, 2H ($CH_2$) ; 3,42 m, 2H ($CH_2$) ; 3,82 s, 3H (OMe) ; 4,77 s, 3H ($COCH_2O$ + NH) ; 6,88-7,28 m, 4H (Ar); 8,38 s, 1H (NH).

**1C- terbutoxycarbamate de la tryptamine 5-(acide O-acétique) :**

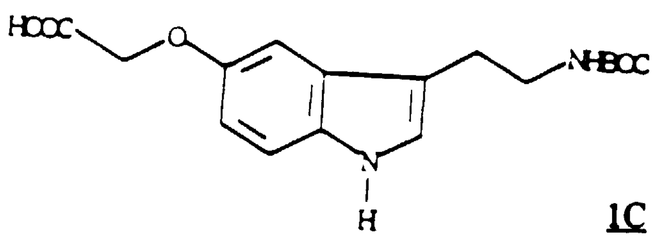

**1C**

Le composé 1B (14,0 g ; 40,24 mmol) en solution dans l'éthanol (250 ml) et l'eau (1 ml) est traité par de la potasse en pastilles (8,9 g ; 157 mmol) à température ambiante pendant 3 heures. Le mélange est ensuite concentré par évaporation, dilué à l'eau, acidifié par de l'acide chlorhydrique (IN) jusqu'à pH 3 et extrait à l'acétate d'éthyle. La phase organique est séchée sur sulfate de sodium, filtrée puis évaporée à sec.
Le sirop jaune obtenu (12,1 g ; 90 %) est utilisé sans autre purification.
Spectre de résonance magnétique nucléaire du proton, $CDCl_3$ (ppm) : 1,44 s, 9H (tBu) ; 2,88 t, 2H ($CH_2$) ; 3,42 m, 2H ($CH_2$) ; 4,70 s, 2H ($COCH_2O$) ; 4,98 s, 1H (NH) ; 6,90-7,30 m, 4H (Ar); 8,05 s, 1H (NH).

**1D - Chlorhydrate de la 4-*o*-tolyl 1-(tertbutoxycarbamate-tryptamine 5-O-carboxyméthyl) pipérazide :**

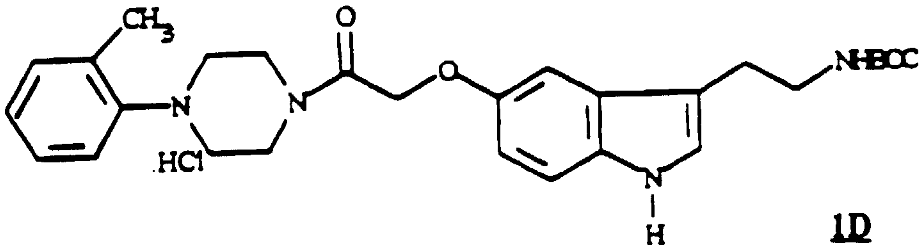

1D

Un mélange du composé 1C (1,0 g ; 2,99 mmol) et de N-méthylmorpholine (0,362 ml ; 3,3 mmol) dans le dichlorométhane à -10°C est traité, goutte à goutte, par le chloroformiate d'éthyle. Après une agitation de 10 minutes, l'ortho-tolylpipérazine (1,1 g ; 4,5 mmol) est additionnée puis le mélange est agité pendant 2 heures de -10°C à température ambiante. Le milieu est ensuite dilué au dichlorométhane, lavé au bicarbonate de sodium puis à l'eau. La phase organique est séchée sur sulfate de sodium, filtrée et évaporée. Le sirop obtenu est chromatographié sur colonne de gel de silice éluée par un mélange dichlorométhane/méthanol (20:1 ; v/v). Le composé pur est isolé sous forme de solide blanc (1,16 g ; 69 %) recristallisé dans l'éther éthylique. Le composé obtenu est dilué dans le dichlorométhane et le chlorhydrate est formé par addition de la quantité nécessaire d'acide chlorhydrique dans l'éther. Les cristaux sont recristallisés dans l'acétate d'éthyle.
Analyse élémentaire ($C_{28}H_{37}N_4O_4Cl$), % calculés : C 63,56 ; H 7,04 ; N 10,59 ; % trouvés : C 64.31 ; H 6.88 ; N 10.65.
Spectre de résonance magnétique nucléaire du proton, DMSO-$d_6$ (ppm) : 1,38 s, 9H (tBu) ; 2,28 s, 3H ($CH_3$) ; 2,79 m, 6H ($CH_2$) ; 3,21 m, 2H ($CH_2$) ; 3,65 s, 2H ($CH_2$) ; 4,80 s, 2H ($COCH_2O$) ; 6,75-7,26 m, 8H (Ar) 10,67 s, 1H (NH).
Point de fusion : 134°C.

**1- Chlorhydrate de la 4-*o*-tolyl 1-(tryptamine 5-O-carboxyméthyl) pipérazide :**

Le composé 1D sous forme de base libre (200 mg ; 0,41 mmol) en solution dans le toluène (10 ml) est traité par l'acide trifluoroacétique (2 ml). Après 1 heure d'agitation à température ambiante le milieu est dilué à l'acétate d'éthyle, lavé à la soude 2N (2 fois), à l'eau puis avec une solution saturée de chlorure de sodium. La phase organique est séchée sur sulfate de sodium, filtrée et évaporée.
Le sirop obtenu est chromatographié sur colonne de gel de silice, élué par un mélange chloroforme/méthanol/ammoniaque (85:14:1, v/v). Le produit pur est isolé sous forme de sirop incolore (92 mg ; 57 %). Le composé obtenu est dilué dans le dichlorométhane et le chlorhydrate est formé par addition de la quantité nécessaire d'acide chlorhydrique dans l'éther.
Analyse élémentaire ($C_{23}H_{34}N_4O_4Cl_2$), % calculés: C 61,53 ; H 6,08 ; N 10,25 ; % trouvés : C 61,25 ; H 6,07 ; N 10,06.
Spectre de résonance magnétique nucléaire du proton, DMSO-$d_6$ (ppm) : 2,30 s, 3H ($CH_3$) ; 2,85-3,00 m, 8H ($CH_2$) ; 3,66 s, 4H ($CH_2$) ; 4,85 s, 2H ($COCH_2O$) ; 6,76-7,29 m, 8H (Ar) ; 8,05 s, 3H ($NH_3^+$) ; 10,86 s, 1H ($NH^+$).
Point de fusion : 147°C.

**Exemple 2 - Chlorhydrate de la 4-*o*-tolyl 1-(tryptamine 5-O-carboxyméthyl) pipérazide :**

METHODE A

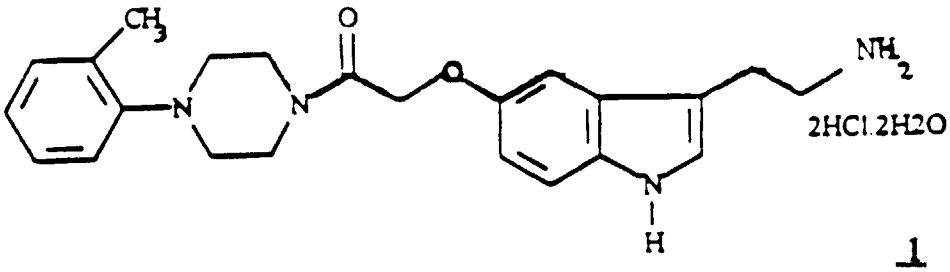

1

**2A - 4-*o*-tolyl-1-chloroacétyl-pipérazide :**

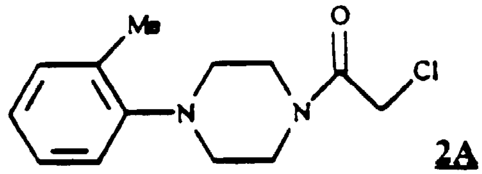

2A

L'ortho-tolyl pipérazine (3,5 g ; 20,0 mmol) en solution dans la méthyléthylcétone (35 ml), en présence de carbonate de calcium (6 g ; 60,0 mmol), est traitée à 0°C goutte à goutte par le chlorure de chloroacétyle (1,59 ml ; 20,0 mmol). Après 15 minutes, le milieu est dilué à l'acétate d'éthyle, filtré sur célite, lavé à l'eau puis par une solution de chlorure de sodium. La phase organique est séchée sur sulfate de sodium, filtrée et évaporée. Le solide jaune obtenu (3,7 g ; 74 %) est engagé sans autre purification dans l'étape suivante.

**1D- Chlorhydrate de la 4-*o*-tolyl 1-(terbutoxycarbamate-tryptamine 5-O-carboxyméthyl) pipérazide :**

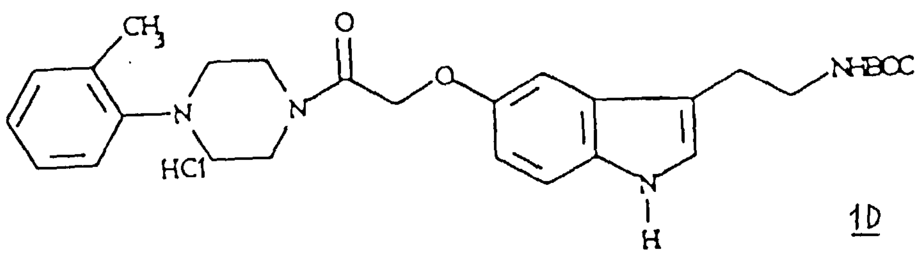

1D

Un mélange du composé 1A (2,27 g ; 8,23 mmol) et du composé 2A (3,7 g ; 14,8 mmol) dans la méthyléthylcétone (45 ml), en présence de carbonate de potassium (2,8 g ; 20,6 mmol) et d'ioduré de potassium (70 mg ; 0,41 mmol) est chauffé au reflux pendant 5 heures. Le milieu est ensuite dilué à l'acétate d'éthyle, filtré sur célite, lavé à la soude (0,5N), à l'eau, puis par une solution saturée de chlorure dc sodium. La phase organique est séchée sur sulfate de sodium, filtrée puis évaporée à sec. Le sirop obtenu est chromatographié sur colonne de gel de silice éluée par un mélange dichlorométhane/acétate d'éthyle (4:1 ; v/v). Le produit pur est isolé sous forme de sirop incolore (3,16 g ; 78 % pour les 2 étapes).
Ce produit est dilué dans le dichlorométhane et le chlorhydrate est formé par addition de la quantité nécessaire d'acide chlorhydrique dans l'éther. Les cristaux sont recristallisés dans l'acétate d'éthyle.
Les caractéristiques structurales et physiques sont décrites dans l'exemple 1D.

**1- Chlorhydrate de la 4-*o*-tolyl 1-(tryptamine 5-O-carboxyméthyl) pipérazide :**

Le produit 1D (3,2 g ; 6,61 mmol) conduit au produit 1 (1,7 g ; 67%) par la méthode préalablement décrite pour la synthèse du produit 1 à partir de 1D. Le produit 1 obtenu possède les mêmes caractéristiques structurales et physiques que l'exemple 1.

**Exemple 3 - Chlorhydrate de la 4-(α, α, α,-trifluoro-m-tolyl) 1-(tryptamine 5-O-carboxyméthyl) pipérazide :**

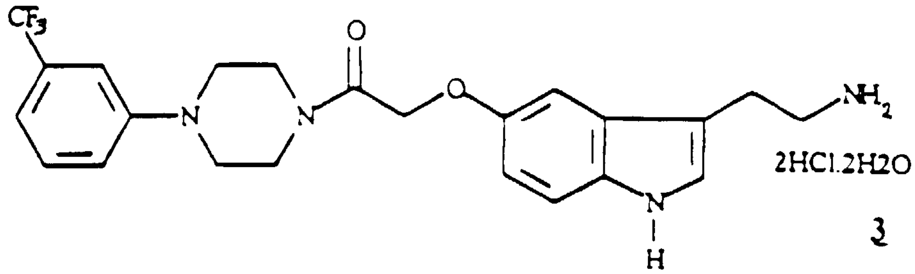

2HCl.2H2O

3

Le composé 3 est obtenu à partir de la 1-(α, α, α,-trifluoro-m-tolyl) pipérazine (0,338 ml ; 1,8 mmol), de chlorure de chloroacétyle (0,143 ml ; 1,8 mmol) et du terbutoxycarbamate de la 5-hydroxytryptamine 1A (276 mg ; 1,0 mmol) selon la procédure décrite pour la préparation de l'exemple 2. La purification du produit sous forme de base est effectuée par chromatographie sur colonne de gel de silice éluée par un mélange chloroforme/méthanol/ammoniaque (85:14: 1 ; v/v). Le produit pur est isolé sous forme de sirop incolore qui conduit, après traitement à l'acide chlorhydrique dans l'éther, au dichlorohydrate dihydrate du composé 3 (258 mg ; 58%).
Analyse élémentaire ($C_{23}H_{31}Cl_2F_3N_4O_4$), % calculés : C 49,73 ; H 5,62 ; N 10,08 ; % trouvés : C 49,73 ; H 5,12 : N 9,94.
Spectre de résonance magnétique nucléaire du proton, DMSO-$d_6$ (ppm) : 2,99 m, 4H ($CH_2$) ; 3,30 m, 4H ($CH_2$) ; 3,67 m, 4H ($CH_2$) ; 4,83 s, 2H ($COCH_2O$) ; 6,77-7,49 m, 8H (Ar) ; 8,01 s, 3H ($NH_3^+$) ; 10,86 s, 1H (NH).
Point de fusion : 131°C.

**Exemple 4 - Chlorhydrate de la 4-(napht-1-yl) 1-(tryptamine 5-O-carboxyméthyl) pipérazide :**

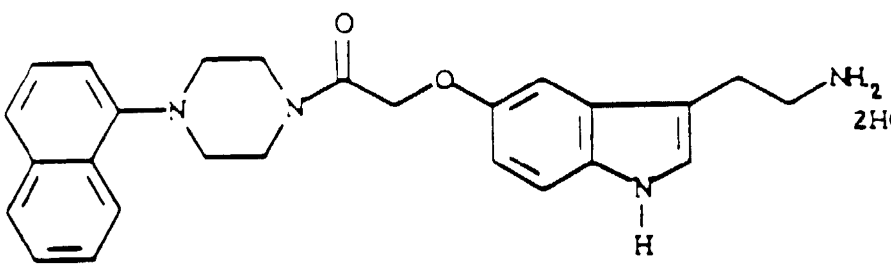

2HCl . 1/3 Et2O

4

Le composé 4 est obtenu à partir de la 1-naphtylpipérazine (478 mg ; 2,25 mmol), de chlorure de chloroacétyle (0,179 ml ; 2,25 mmol) et du terbutoxycarbamate de la 5-hydroxytryptamine 1A (341 mg ; 1,24 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19:1 ; v/v). Le produit pur est isolé sous forme de sirop incolore qui conduit, après traitement à l'acide chlorhydrique dans l'éther au composé 4.
Analyse élémentaire ($C_{26}H_{30}Cl_2N_4O_2$, 1/3 $Et_2O$), % calculés : C 62,34 ; H 6,39 ; N 10,65 ; % trouvés : C 62.52 ; H 6.49 ; N 10.45.
Spectre de résonance magnétique nucléaire du proton, DMSO-$d_6$ (ppm) : 1,09 t, 2H (1/3 Et) ; 3,00 s, 8H ($CH_2$) ; 3,36-q, 4/3 H (1/3 Et) ; 3,84 s, 4H ($CH_2$) ; 4,85 s, 2H ($COCH_2O$) ; 6,80-8,22 m, 14H (Ar + $NH_3^+$); 10,86 s, 1H (NH).
Point de fusion : 220°C.

**Exemple 5 - Chlorhydrate de la 4-(2,3-xylyl) 1-(tryptamine 5-O-carboxyméthyl) pipérazide :**

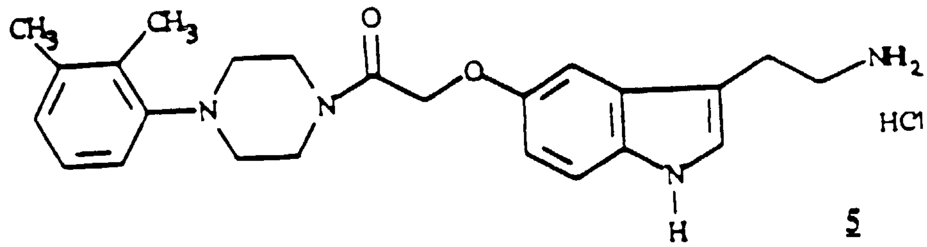

HCl

5

**5A - Chlorhydrate de la 4-(2,3-xylyl) 1-(terbutoxycarbamate-tryptamine 5-O-carboxyméthyl) pipérazide :**

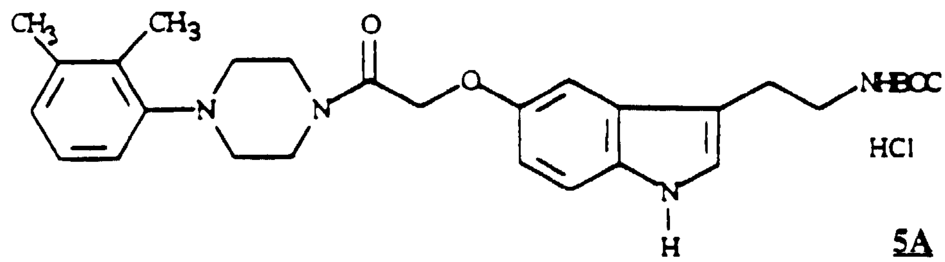

HCl

5A

Le composé 5A est obtenu à partir de la 1-(2,3-xylyl)-pipérazine (343 mg ; 1,8 mmol), de chlorure de chloroacétyle (0,143 ml ; 1,8 mmol) et du terbutoxycarbamate de la 5-hydroxytryptamine 1A (276 mg ; 1,0 mmol) selon la procédure décrite pour la préparation du composé 2B. La purification du produit sous forme de base est effectuée par chromatographie sur colonne de gel de silice éluée par un mélange dichlorométhanelacétone (20:1 ; v/v) puis (10:1 ; v/v). Le produit pur est isolé sous forme de sirop incolore qui conduit, après traitement à l'acide chlorhydrique dans l'éther au composé attendu.
Analyse élémentaire ($C_{29}H_{39}N_4O_4Cl$), % calculés : C 64,13 ; H 7,24 ; N 10,32 ; % trouvés : C 63.43 ; H 7.35 ; N 10.01.
Spectre de résonance magnétique nucléaire du proton, DMSO-$d_6$ (ppm) : 1,37 s, 9H (tBu) ; 2,21 s, 6H ($CH_3$) ; 2,75 m, 6H ($CH_2$) ; 3,18 s, 2H ($CH_2$) ; 3,68 s, 4H ($CH_2$) ; 4,80 s, 2H ($COCH_2O$) ; 6,74-7,25 m, 7H (Ar); 10,68 s, 1H (NH).
Point de fusion : 90°C.

**5 - Chlorhydrate de la 4-(2,3-xylyl) 1-(tryptamine 5-O-carboxyméthyl) pipérazide :**

Le composé 5A, sous forme de base (173 mg ; 0,34 mmol), traité dans les conditions décrites pour la préparation du produit 1 à partir du produit 1D et purifié dans les mêmes conditions, permet d'obtenir un sirop incolore (108 mg ; 78 %).
Ce sirop conduit au bis chlorhydrate 5 par addition de la quantité nécessaire d'acide chlorhydrique dans l'éther.
Analyse élémentaire ($C_{24}H_{32}Cl_2N_4O_2$, 1/3 $Et_2O$), % calculés : C 60,12 ; H 6,73 ; N 11,69; % trouvés : C 61.22; H 7.07 ; N 11.31.
Spectre de résonance magnétique nucléaire du proton, DMSO-$d_6$ (ppm) : 2,22 s, 6H ($CH_3$) ; 2,81-3,00 m, 8H ($CH_2$) ; 3,70 s, 4 H ($CH_2$) ; 4,82 s, 2H ($OCH_2CO$) ; 6,77-7,29 m, 7H (Ar) ; 8,08 s, 3H ($NH_3^+$) ; 10,86 s, 1H (NH).
Point de fusion : 132°C.

**Exemple 6 - Chlorhydrate de la 4-(m-méthoxyphenyl) 1-(tryptamine 5-O-carboxyméthyl) pipérazide :**

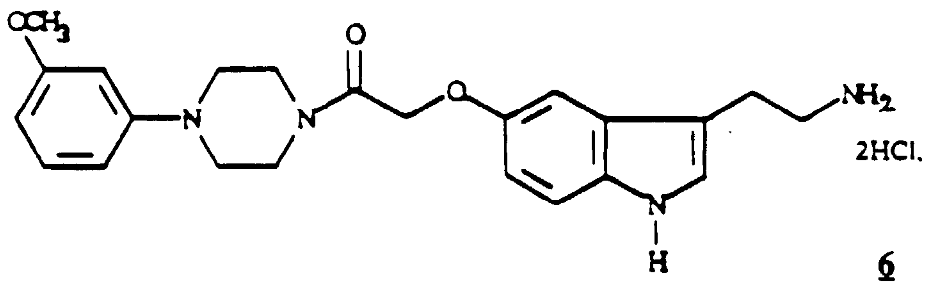

2HCl. $2H_2O$

6

**6A - Chlorhydrate de la 4-(m-méthoxyphényl) 1-(terbutoxycarbamate-tryptamine 5-O-carboxyméthyl) pipérazide :**

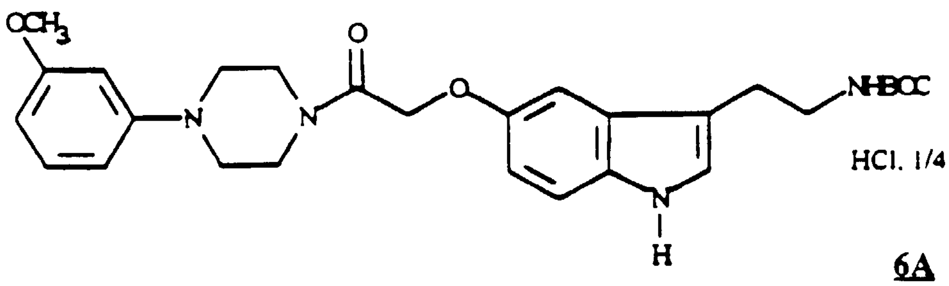

HCl. 1/4 $Et_2O$

6A

Le composé 6A est obtenu à partir de la m-méthoxyphényl pipérazine (300 mg ; 1,56 ml), de chlorure de chloroacétyle (0,124 ml ; 1,56 mmol) et du terbutoxycarbamate de la 5-hydroxytryptamine 1A (237 mg ; 0,86 mmol) selon la procédure décrite pour la préparation du composé 2B (méthode A). La purification du produit sous forme de base est effectuée par chromatographie sur colonne de gel de silice éluée par un mélange dichlorométhane/acétone (10:1, v/v). Le produit pur est isolé sous forme de sirop incolore qui conduit, après traitement à l'acide chlorhydrique dans l'éther au composé attendu (707 mg ; 78 %).
Analyse élémentaire ($C_{28}H_{37}ClN_4O_5$, 1/4 $Et_2O$), % calculés : C 59,89 ; H 7,19 ; N 9,63 ; % trouvés : C 59.97 ; H 6.87 ; N 9.62.
Spectre de résonance magnétique nucléaire du proton, DMSO-$d_6$ (ppm) : 1,09 t, 3/2 H ($Et_2O$) ; 1,37 s, 9H (tBu) ; 2,74 m, 2 H ($CH_2$) ; 3,32 m, 7H ($CH_2$ + $Et_2O$) ; 3,74 s, 6H ($CH_2$ + $H_2O$) ; 4,81 s, 2H ($OCH_2CO$) ; 6,55-7,25 m, 9H (Ar + NH) ; 10,70 s, 1H (NH).
Point de fusion : 146°C.

**6 - Chlorhydrate de la 1-(tryptamine 5-O-carboxyméthyl) 4-(m-méthoxyphényl) pipérazide :**

Ce composé est obtenu à partir du produit 6A (154 mg ; 0,302 mmol) selon la procédure décrite pour la préparation du produit 1 à partir de 1D. La purification du produit sous forme de base est effectuée par chromatographie sur colonne de gel de silice éluée par un mélange chloroforme/méthanol/ammoniaque (80:19:1 ; v/v). Un sirop incolore est obtenu (111 mg ; 91 %).
Ce sirop conduit au bis-chlorhydrate par addition de la quantité nécessaire d'acide chlorhydrique dans l'éther.
Spectre de résonance magnétique nucléaire du proton, DMSO-$d_6$ (ppm) : 3,00 s, 4H ($CH_2$) ; 3,26 d, 4H ($CH_2$) ; 3,74 s, 7 H ($CH_2$ + $OCH_3$) ; 4,83 s, 2H ($OCH_2CO$) ; 6,54-7,28 m, 8H (Ar) ; 8,16 s, 3H (NH) ; 10,86 s, 1H (NH).

**Exemple 7 - Chlorhydrate de la 4-méthyl 1-(tryptamine 5-O-carboxyméthyl) pipérazide :**

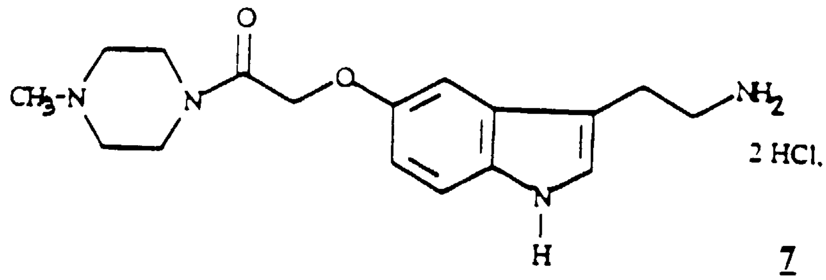

2 HCl. $H_2O$

7

**7A - Chlorhydrate de la 4-méthyl 1-(terbutoxycarbamate-tryptamine 5-O-carboxyméthyl) pipérazide :**

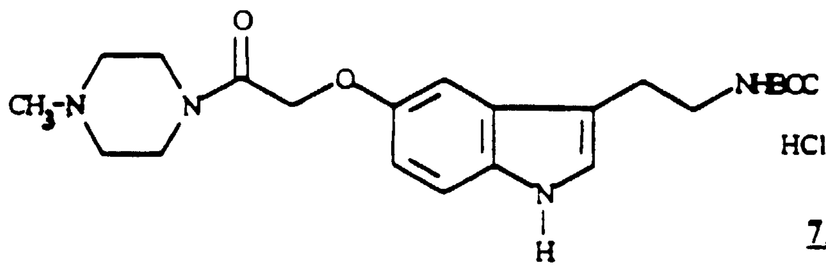

HCl. $H_2O$

7A

Le composé 7A est obtenu à partir de la méthyl pipérazine (300 mg ; 3,00 mmol), de chlorure de chloroacétyle (0,239 ml ; 3,0 mmol) et du terbutoxycarbamate de la 5-hydroxytryptamine 1A (455 mg ; 1,65 mmol) selon la procédure décrite pour la préparation du composé 2B (méthode A). La purification du produit sous forme de base est effectuée par chromatographie sur colonne de gel de silice éluée par un mélange dichlorométhane/méthanol (10:1, v/v). Le produit pur est isolé sous forme de sirop incolore qui conduit, après traitement à l'acide chlorhydrique dans l'éther, au composé attendu (918 mg, 65 %).
Analyse élémentaire ($C_{22}H_{35}ClN_4O_5$), % calculés : C 56,10 ; H 7,49 ; N 11,90 ; % trouvés : C 57.21 ; H 7.50 ; N 11.51.
Spectre de résonance magnétique nucléaire du proton, DMSO-$d_6$ (ppm) : 1,36 s, 9H (tBu) ; 2,75 s, 5H ; 3,21 m, 10H ; 4,80 s, 2H ($OCH_2CO$) ; 6,74-7,25 m, 5H (Ar + NH) ; 10,69 s, 1H (NH) ; 11,28 s, 1H (NH).
Point de fusion : 204°C.

**7 - Chlorhydrate de la 4-méthyl 1-(tryptamine 5-O-carboxyméthyl) pipérazide:**

Ce composé est obtenu à partir du composé 7A (150 mg ; 0,36 mmol) selon la procédure décrite pour la préparation du produit 1 à partir du produit 1D. La purification du produit sous forme de base est effectuée par chromatographie sur colonne de gel de silice éluée par un mélange chloroforme/méthanol/ammoniaque (80:19:1 ; v/v). Un sirop incolore est obtenu (102 mg ; 79 %).
Le bis-chlorhydrate est obtenu par addition de la quantité nécessaire d'acide chlorhydrique dans l'éther.
Spectre de résonance magnétique nucléaire du proton, DMSO-$d_6$ (ppm) : 2,77 s, 3H 3,00-3,57 m, 10H ($CH_2$) ; 4,15-4,37 m, 2H ; 4,85 s, 2H ($COCH_2$) ; 6,80 dd, 1H (Ar) ; 7,23 m, 3H (Ar) ; 8,18 s, 3H ($NH_3^+$) ; 10,86 s, 1H (NH) ; 11,39 s, 1H (NH).

**Exemple 8 - Chlorhydrate de la 1-[(terbutoxycarbamate)-tryptamine 5-0-carboxyméthyl]-4-[10-(5-méthylthio-10-11-dihydrodibenzo[b,f]thiépine)] pipérazide:**

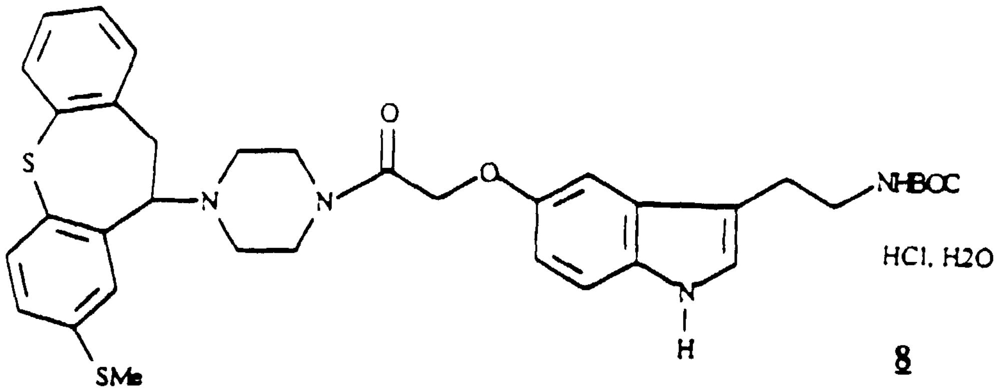

8

Ce composé est obtenu à partir du 8-méthylthio-10-pipérazino-10-11-dihydrodibenzo [b,f]thiépin (Protiva et Col., Collection Czechoslov. Chem. Commun., 36, 2226-2247, 1971) (300 mg, 0,876 mmol), de chlorure de chloroacétyle (0,069 ml ; 0,876 mmol) et du terbutoxycarbamate de la 5-hydroxytryptamine 1A (132 mg ; 0,48 mmol) selon la procédure décrite pour la préparation du composé 2B (méthode A). La purification du produit sous forme de base est effectuée par chromatographie sur colonne de gel de silice éluée par un mélange dichlorométhane/acétate d'éthyle (5:1, v/v). Le produit pur est isolé sous forme de sirop incolore (420 mg ; 74 %). Le chlorhydrate est obtenu par addition de la quantité nécessaire d'acide chlorhydrique dans l'éther.
Analyse élémentaire ($C_{36}H_{43}N_4O_4S_2Cl$), % calculés : C 60,61 ; H 6,35 ; N 7,85; % trouvés : C 61.15 ; H 6.65 ; N 7.52.
Spectre de résonance magnétique nucléaire du proton, DMSO-$d_6$ (ppm) : 1,37 s, 9H (tBu) ; 2,74 m, 2H ; 3,17-3,99 m, 13H ($CH_2$) ; 4,78 s, 2H ($OCH_2CO$) ; 5,26 s, 1H (Ar) ; 10,69 s, 1H (NH) ; 11.46 s, 1H (NH).
Point de fusion : 158°C.

**Exemple 9 - Chlorhydrate de la 4-O-tolyl 1-[tryptamine 5-0 (α-phénylcarboxyméthyl)] pipérazide :**

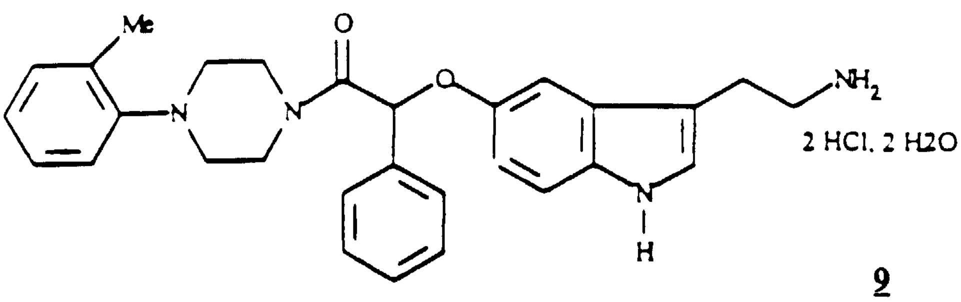

9

Le composé 9 est obtenu à partir de la 4-o-tolyl pipérazine (2,5 g ; 14,3 mmol), du chlorure d'α-chloro-phénylacétyl (2,26 ml ; 14,3 mmol) et du terbutoxycarbamate de la 5-hydroxytryptamine 1A (2,17 g ; 7,86 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19/1, v/v). Le produit pur est isolé sous forme de sirop incolore (58 %). Le chlorhydrate est obtenu par addition de la quantité nécessaire d'acide chlorhydrique dans l'éther.
Analyse élémentaire ($C_{29}H_{36}N_4O_3Cl_2$), % calculés : C 63,79 ; H 6,50 ; N 10,26; % trouvés : C 63.83 ; H 6.61 ; N 10.00
Spectre de résonance magnétique nucléaire du proton, DMSO-$d_6$ (ppm) : 2,24 s, 3H ($CH_3$) ; 2,75 m, 4H ($CH_2$) ; 2,99 s, 4H ($CH_2$) ; 3,47-3,86 m, 4H ($CH_2$) ; 6,34 s, 1H (CH) ; 6,83-7,62 m, 13H (Ar) ; 8,11 s, 3H ($NH_3^+$) ; 10,88 s, 1H (NH).
Point de fusion : 164°C.

**Exemple 10 - Chlorhydrate de la 4-naphtyl 1-[tryptamine 5-0 ($\alpha$-phénylcarboxyméthyl)] pipérazide :**

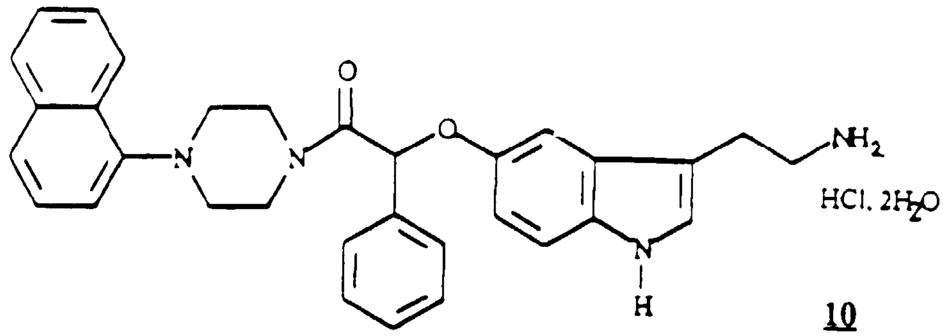

**10**

Le composé 10 est obtenu à partir de la napht-1-yl pipérazine (0,60 g ; 4,18 mmol), du chlorure d'$\alpha$-chloro-phénylacétyl (0,66 ml ; 4,18 mmol) et du terbutoxycarbamate de la 5-hydroxytryptamine 1A (0,63 g ; 2,30 mmol) selon la procédure décrite pour la préparation de l'exemple 2. Le produit, sous forme de base, est purifié par chromatographie sur colonne de gel de silice éluée par un mélange chloroforme/méthanol/ammoniaque (80:19/1, v/v). Le produit pur est isolé sous forme de sirop incolore (1,03 g ; 49 % pour les 3 étapes). Le chlorhydrate est obtenu par addition de la quantité nécessaire d'acide chlorhydrique dans l'éther.
Analyse élémentaire ($C_{32}H_{37}N_4O_4Cl$), % calculés : C 66,54 ; H 6,41 ; N 9,70; % trouvés : C 67,02 ; H 6,16 ; N 9.33
Spectre de résonance magnétique nucléaire du proton, DMSO-$d_6$ (ppm) : 2,99 s, 8H ($CH_2$) ; 4,00 m, 4H ($CH_2$) ; 8,36 s, 1H (CH) ; 6,85-7,87 m, 16H (Ar) ; 8,13 s, 3H ($NH_3^+$) ; 10,89 s, 1H (NH).
Point de fusion : 178°C.

**Exemple 11 - Chlorhydrate de la 4-o-tolyl 1-[tryptamine 5-0 ($\alpha$-méthylcarboxyméthyl)] pipérazide :**

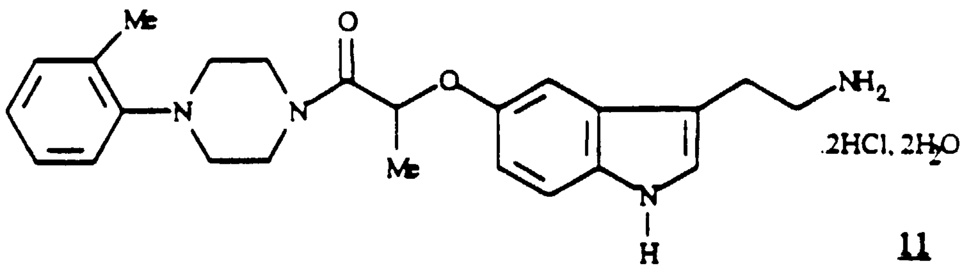

**11**

Le composé 11 est obtenu à partir de la 4-o-tolyl pipérazine (0.40 g ; 2,26 mmol), du chlorure d'$\alpha$-méthyl-chloroacétyle (0,22 ml ; 2,26 mmol) et du terbutoxycarbamate de la 5-hydroxytryptamine 1A (0,34 g ; 1,24 mmol) selon la procédure décrite pour la préparation de l'exemple 2. Le produit 11, sous forme de base, est purifié par chromatographie sur colonne de gel de silice éluée par un mélange chloroforme/méthanol/ammoniaque (80:18,5:1, v/v). Le produit pur est isolé sous forme de sirop incolore (269 mg ; 30 % pour les 3 étapes). Le chlorhydrate est obtenu par addition de la quantité nécessaire d'acide chlorhydrique dans l'éther.
Analyse élémentaire ($C_{24}H_{36}N_4O_4Cl_2$), % calculés : C 55,92 ; H 7,03 ; N 10,86 ; % trouvés : C56,54 ; H 6,83 ; N 10,62.
Spectre de résonance magnétique nucléaire du proton, DMSO-$d_6$ (ppm) : 1,45 d, 3H ($CH_3$) ; 2,28 s, 3H ($CH_3$) ; 2,81 s, 4H ($CH_2$) ;2,99 s, 4H ($CH_2$) ;3,62-3,77 m, 4H ($CH_2$) ; 5,28 q, 1H (CH) ; 6,75 dd, 1H ; 6,95-7,29 m, 7H (Ar) ; 8,09 s, 3H ($NH_3^+$) ; 10,88 s, 1H (NH).
Point de fusion : 153-154°C (polymorphisme).

**Exemple 12 - Chlorhydrate de la 4-(napht-1-yl) 1-(tryptamine 5-O-méthylsulfonyl) pipérazide :**

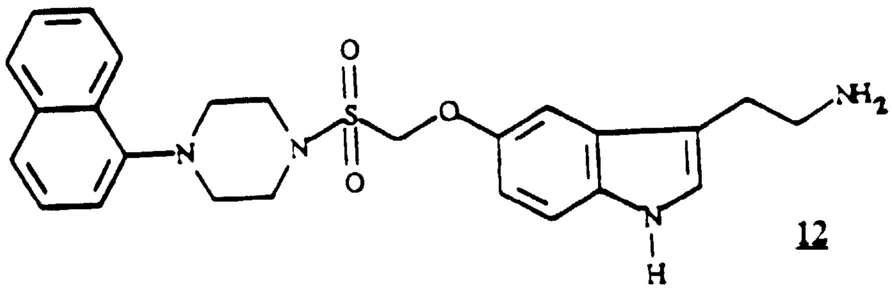

12

Le composé 12 est obtenu à partir de la 1-naphtylpipérazine (800 mg ; 3,76 mmol), de chlorure de chlorométhanesulfonyl (561 mg ; 3,76 mmol) et du terbutoxycarbamate de la 5-hydroxytryptamine 1A (520 mg ; 2,07 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19: 1 ; v/v). Le produit pur est isolé sous forme de sirop incolore qui conduit, après traitement à l'acide chlorhydrique dans l'éther au composé attendu (580 mg ; 56%).

**Exemple 13 - Chlorhydrate de la 4-*m*-chlorophenyl 1-(tryptamine 5-O-propyl) pipérazine :**

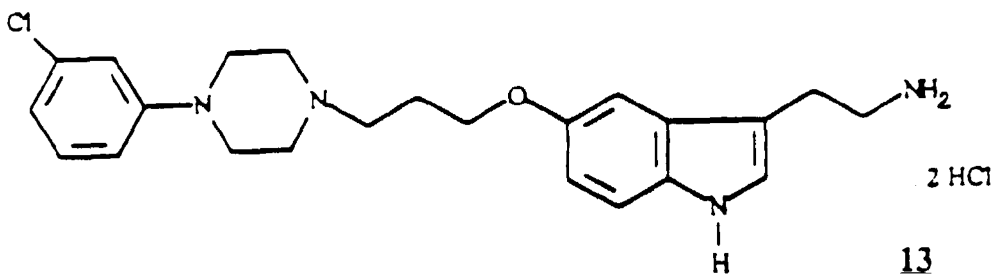

13

**13A - 4-*m*-chlorophenyl 1-(terbutoxycarbamate-tryptamine 5-O-propyl) pipérazine :**

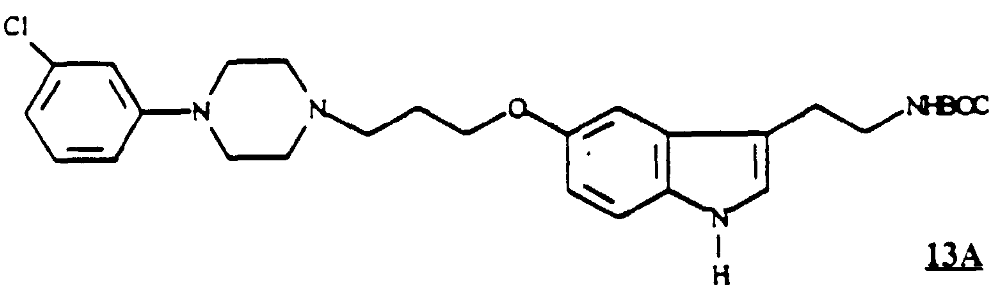

13A

Un mélange de la 1-(3-chlorophényl)-4-(3-chloropropyl) pipérazine (700 mg ; 2,56 mmol) et du terbutoxycarbamate de la 5-hydroxytryptamine 1A (353 mg ; 1,28 mmol) dans le diméthylformamide (6 ml) en présence d'iodure de potassium (425 mg ; 2,56 mmol) et de carbonate de potassium (530 mg ; 3,84 mmol) est chauffé à 100°C pendant 7 heures. Le milieu est ensuite dilué à l'éther, lavé à l'eau puis par une solution saturée de chlorure de sodium. La phase organique est séchée sur sulfate de sodium, filtrée puis évaporée à sec. Le sirop obtenu est chromatographie sur colonne de gel de silice éluée par un mélange dichlorométhane/méthanol (20/1 ; v/v). Le composé pur est isolé sous forme de sirop (426 mg; 65%).

**13 - Chlorhydrate de la 4-*m*-chlorophenyl 1-(tryptamine 5-O-propyl) pipérazine :**

Le composé 13A (200 mg; 0,39 mmol), traité dans les conditions décrites pour la préparation du produit 1 à partir du produit 1D et purifié dans les mêmes conditions permet d'obtenir un sirop incolore (133 mg; 75%). Ce sirop conduit au chlorhydrate 13 par addition de la quantité nécessaire d'acide chlorhydrique dans l'éther.

**Exemple 14 - Chlorhydrate de la 4-(4-acétyl-phényl) 1-(tryptamine 5-O-carboxyméthyl) pipérazide :**

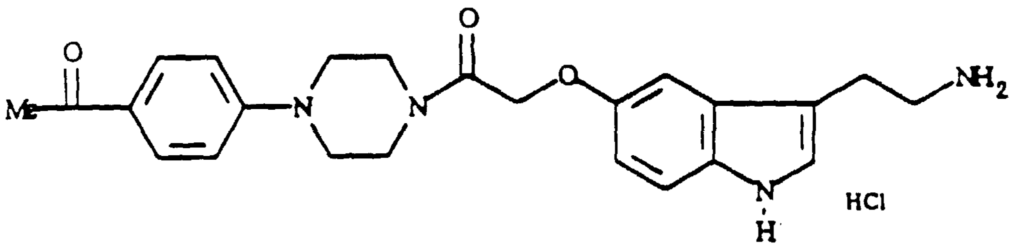

HCl

**14**

Le composé 14 est obtenu à partir de la 4'-pipérazinoacétophénone (531 mg ; 2,44 mmol), de chlorure de chloroacétyle (0,194 ml; 2,44 mmol) et du terbutoxycarbamate de la 5-hydroxytryptamine 1A (374 mg; 1,35 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19:1; v/v). Le produit pur est isolé sous forme de sirop jaune qui conduit, après traitement à l'acide chlorhydrique dans l'éther, au composé attendu (307 mg; 45%).
Analyse élémentaire ($C_{24}H_{29}N_4O_3Cl$), % calculés : C 63,08; H 6,40; N 12,26; % trouvés : C 62,86; H 6,55; N 12,03.
Spectre de résonnance magnétique nucléaire du proton, DMSO-$d_6$ (ppm) : 2,46 s, 3H ($CH_3$); 2,92-3,02 m, 4H ($CH_2$); 3,37 m, 4H ($CH_2$); 3,63 m, 4H ($CH_2$); 4,83 s, 2H ($COCH_2O$); 6,77-7,29 m, 6H (Ar); 7,69-7,85 m, 5H (Ar + $NH_3$+); 10,85 s, 1H (NH).
Point de fusion : 155°C (déc.).

**Exemple 15 - Chlorhydrate de la 4-(2,4-diméthyl-phényl) 1-(tryptamine 5-O-carboxyméthyl) pipérazide :**

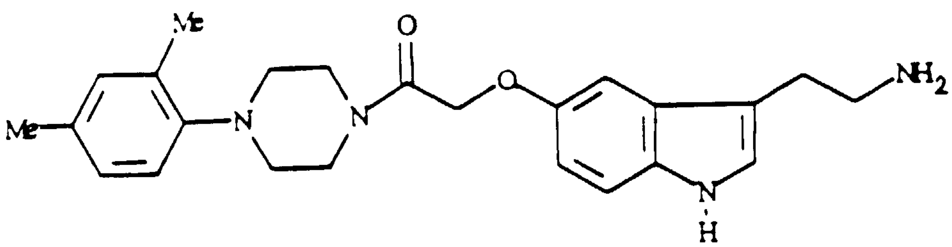

2HCl

**15**

Le composé 15 est obtenu à partir de la 1-(2,4-diméthyl-phényl) pipérazine (1000 mg; 5,26 mmol), de chlorure de chloroacétyle (0,419 ml); 5,26 mmol) et du terbutoxycarbamate de la 5-hydroxytryptamine 1A (682 mg; 2,47 mmol) selon la procédure décrite pour la préparation de l'exemple 2. La purification du produit sous forme de base est effectuée par chromatohraphie sur colonne de gel de silice éluée par un mélange chloroforme/méthanol/ammoniaque (80:18,2; v/v). Le produit pur est isolé sous forme de sirop incolore qui conduit, après traitement à l'acide chlorhydrique dans l'éther, au composé attendu (720 mg; 71%).
Analyse élémentaire ($C_{24}H_{32}N_4O_2Cl_2$), % calculés : C 60,12; H 6,73; N 11,69; % trouvés : C 59,85; H 6,99; N 11,58.
Spectre de résonnance magnétique nucléaire du proton, DMSO-$d_6$ (ppm): 2,23 s, 3H ($CH_3$); 2,29 s, 3H ($CH_3$); 2,89-3,00 m, 8H ($CH_2$); 3,70 m, 4H ($CH_2$); 4,83 s, 2H ($COCH_2O$); 6,77-7,29 m, 7H (Ar); 8,11 s, 3H ($NH_3$+); 10,87-10,88 d, 1H (NH).
Point de fusion : 162°C.

**Exemple 16 - Chlorhydrate de la 4-(4-chloro-phényl) 1-(tryptamine 5-O-carboxyméthyl) pipérazide :**

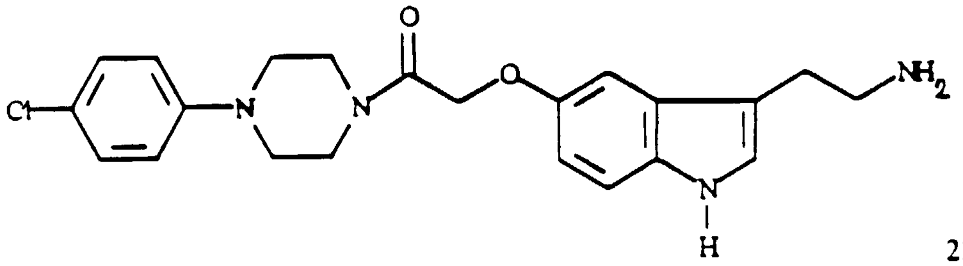

2HCl

**16**

Le composé 16 est obtenu à partir de la 1-(4-chloro-phényl) pipérazine (2,5g; 12,71 mmol), de chlorure de chloroacétyle (1,25 ml; 15,26 mmol) et du terbutoxycarbamate de la 5-hydroxytryptamine 1A (1,53 g; 5,55 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19:1; v/v). Le produit pur est isolé sous forme de sirop incolore qui conduit, après traitement à l'acide chlorhydrique dans l'éther, au composé attendu (1,48 g; 55%).

Analyse élémentaire ($C_{22}H_{27}N_4O_2Cl_3$), % calculés : C 54,39; H 5,60; N 11,53; % trouvés: C 53,96; H 5,58; N 11,11.

Spectre de résonance magnétique nucléaire du proton, DMSO-$d_6$ (ppm) : 2,99-3,22 m, 8H ($CH_2$); 3,67 m, 4H ($CH_2$); 4,83 s, 2H($COCH_2O$); 6,77-7,30 m, 8H(Ar); 8,10 s, 3H ($NH_3^+$); 10,86 s, 1H (NH).

Point de fusion : 259°C.

**Exemple 17 - Chlorhydrate de la 4-(3,4-dichloro-phényl) 1-(tryptamine 5-O-carboxyméthyl) pipérazide :**

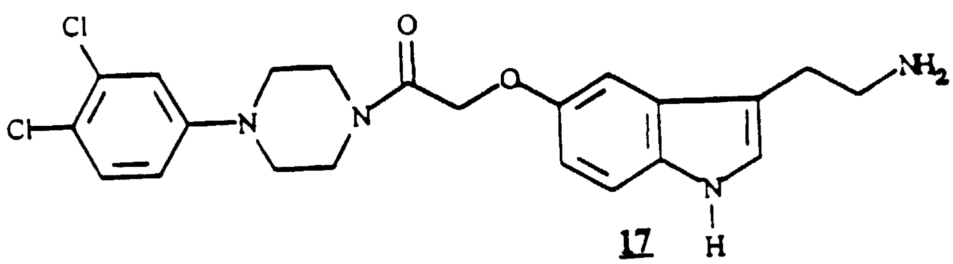

**17** 2HCl

Le composé 17 est obtenu à partir de la 1-(3,4-dichloro-phényl) pipérazine (600 mg; 2,60 mmol), de chlorure de chloroacétyle (0,207 ml; 2,60 mmol) et du terbutoxycarbamate de la 5-hydroxytryptamine 1A (420 mg; 1,52 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19,1; v/v). Le produit pur est isolé sous forme de sirop incolore qui conduit, après traitement à l'acide chlorhydrique dans l'éther, au composé attendu (584 mg; 74%).

Analyse élémentaire ($C_{22}H_{26}N_4O_2Cl_4H_2O$), % calculés : C 48,44; H 5,32; N 10,27; % trouvés : C 48,53; H 5,02; N 9,98.

Spectre de résonnance magnétique nucléaire du proton, DMSO-$d_6$ (ppm) : 3,00-3,27 m, 8H ($CH_2$); 3,65 m, 4H ($CH_2$); 4,82 s, 2H($COCH_2O$); 6,77-7,44 m, 7H(Ar); 8,15 s, 3H ($NH_3$+); 10,86 s, 1H (NH).

Point de fusion : 179-181°C.

**Exemple 18 - Chlorhydrate de la 4-(4-méthoxy-phényl) 1-(tryptamine 5-O-carboxyméthyl) pipérazide :**

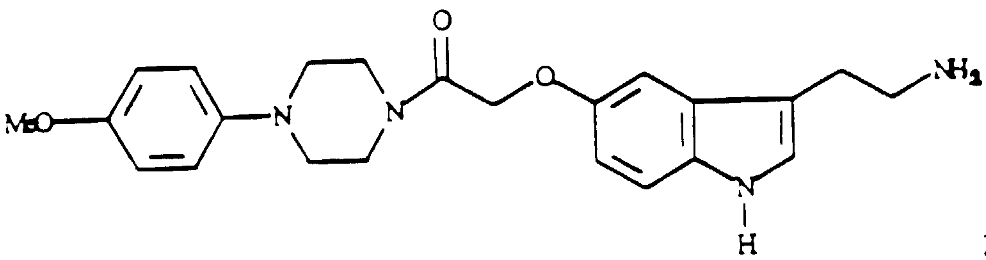

2HCl

**18**

Le composé 18 est obtenu à partir de la 1-(4-méthoxy-phényl) pipérazine (600 mg; 3,12 mmol), de chlorure de chloroacétyle (0,25 ml; 3,12 mmol) et du terbutoxycarbamate de la 5-hydroxytryptamine 1A (362 mg; 1,31 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19,5: 0,5; v/v). Le produit pur est isolé sous forme de sirop incolore qui conduit, après traitement à l'acide chlorhydrique dans l'éther, au composé attendu (455 mg; 53%).

Analyse élémentaire ($C_{23}H_{30}N_4O_3Cl_2$.1,7 $H_2O$), % calculés : C 53,95; H 6,57; N 10,94; % trouvés : C 54,41; H 6,29; N 10,47.

Spectre de résonnance magnétique nucléaire du proton, DMSO-$d_6$ (ppm) : 3,00 s, 4H ($CH_2$); 3,38 m, 4H ($CH_2$); 3,76 s, 3H (OMe); 3,95 m, 4H($CH_2$); 4,87 s, 2H ($COCH_2O$); 6,78-7,53 m, 8H (Ar); 8,09 s, 3H ($NH_3^+$); 10,86 s, 1H (NH).

Point de fusion : 170°C.

**Exemple 19 - Chlorhydrate de la 4-cyclopentyl 1-(tryptamine 5-0-carboxyméthyl) pipérazide :**

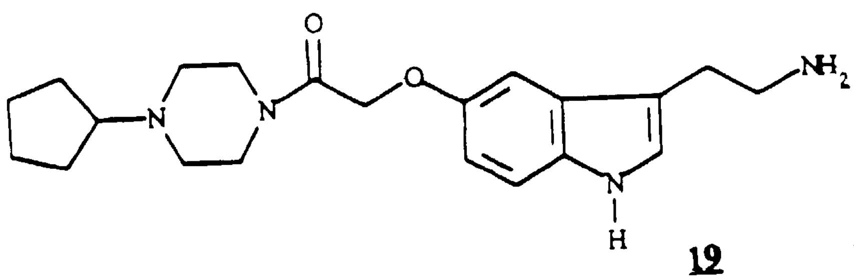

2HCl

**19**

Le composé 19 est obtenu à partir de la 1-cyclopentyl-pipérazine (1000 mg; 6,48 mmol), de chlorure de chloroacétyle (0,516 ml; 6,48 mmol) et du terbutoxycarbamate de la 5-hydroxytryptamine 1A (702 mg; 2,54 mmol) selon la procédure décrite pour la préparation de l'exemple 2. La purification du produit sous forme de base est effectuée par chromatographie sur colonne de gel de silice éluée par un mélange chloroforme/méthanol/ammoniaque (80:19:1; v/v). Le produit pur est isolé sous forme de sirop incolore qui conduit, après traitement à l'acide chlorhydrique dans l'éther, au composé attendu (467 mg; 41%).

Analyse élémentaire ($C_{21}H_{32}N_4O_2Cl_2$.0,7 $H_2O$), % calculés : C 55,31; H 7,38; N 12,29; % trouvés : C 55,44; H 7,03; N 12,03.

Spectre de résonnance magnétique nucléaire du proton, DMSO-$d_6$ (ppm) : 1,42-2,10 m, 8H ($CH_2$); 2,99-3,63 m, 11H; 4,12-4,38 m, 2H; 4,85 s, 2H($COCH_2O$); 6,78-7,29 m, 4H(Ar); 8,05 s, 3H ($NH_3^+$); 10,85 s, 1H (NH); 11,32 s, 1H.

Point de fusion : 195°C(déc.).

**Exemple 20 - chlorhydrate de la 4-(o-tolyl) 1-(tryptamine 5-O-éthyl) pipérazine :**

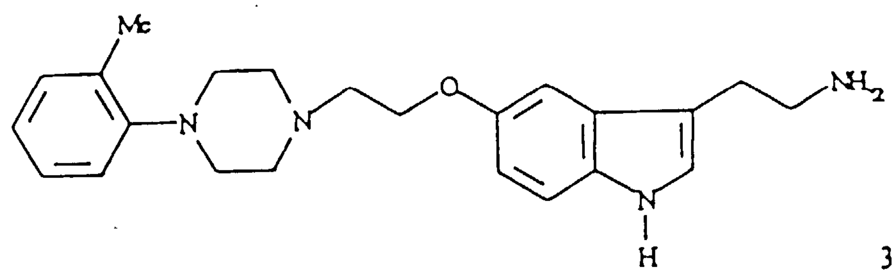

3HCl

20

**20A - terbutoxycarbamate de la 5-(2-chloro-éthoxy)-tryptamine :**

Le produit 1A (5g; 18,09 mmol) en solution dans la méthyléthylcétone (25 ml), en présence de carbonate de potassium (15 g; 108,5 mmol), est traité par le 1-bromo-2-chloro-éthane. Après 24 heures à reflux le milieu est dilué au dichlorométhane, filté sur célite et évaporé à sec. Le sirop brun obtenu est chromatographié sur colonne de gel de silice éluée par un mélange dichlorométhane/acétone (30:1, v/v). Le produit pur est obtenu sous forme de cristaux blancs (5,2 g; 84%).
Analyse élémentaire ($C_{17}H_{23}N_2O_3Cl_3$), % calculés : C 60,26; H 6,84; N 8,27; % trouvés : 60,37; H 6,98; N 8,21.
Spectre de résonnance magnétique nucléaire du proton, DMSO-$d_6$ (ppm) : 1,46 s, 9H(tBu); 2,88-2,95 t, 2H ($CH_2$); 3,45 m, 2H ($CH_2$); 3,81-3,87 t, 2H ($CH_2$); 4,26-4,32 t, 2H ($CH_2$); 4,65 s, 1H (NH); 6,87-6,93 dd, 1H (Ar); 7,01-7,29 m, 3H (Ar); 8,16 s, 1H (NH).
Point de fusion : 129°C.

**20 - chlorhydrate de la 4-(o-tolyl) 1-(tryptamine 5-O-éthyl) pipérazine :**

Un mélange du produit 20A (576 mg; 1,70 mmol) et d'ortho-tolylpipérazine (300 mg; 1,70 mmol) dans le diméthylformamide (0,8 ml) en présence de carbonate de potassium (705 mg, 5,1 mmol) et d'iodure de potassium (28 mg; 0,17 mmol) est chauffé à 80°C pendant 27 heures. Le milieu est alors dilué à l'acétate d'éthyle, filtré sur coton, lavé à l'eau puis par une solution saturée de chlorure de sodium. La phase organique est séchée sur sulfate de sodium, filtrée et évaporée à sec. Le sirop obtenu est purifié sur colonne de gel de silice éluée par un mélange dichlorométhane/méthanol (30:1, v/v). Le produit pur est isolé sous forme de mousse beige (676 mg; 83%). Ce produit est déprotégé selon la méthode décrite pour la préparation de l'exemple 1 à partir de 1D. Le sirop obtenu est chromatographié sur colonne de gel de silice éluée par un mélange dichlorométhane/méthanol/ammoniaque (80:19:1; v/v).
Le produit pur est isolé sous forme de sirop incolore qui conduit, après traitement à l'acide chlorhydrique dans l'éther, au composé 20 (644 mg; 89%).
Analyse élémentaire ($C_{23}H_{33}N_4O_1Cl_3$), % calculés : C 56,62; H 6,82; N 11,48; % trouvés : C 56,53; H 6,99; N 11,16.
Spectre de résonnance magnétique nucléaire du proton, DMSO-$d_6$ (ppm) : 2,27 s, 3H (Me); 3,02-3,37 m, 6H ($CH_2$); 3,63-3,68 m, 4H ($CH_2$); 4,03 s, 4H ($CH_2$); 4,51 m, 2H ($CH_2$); 6,80-7,32 m, 8H (Ar); 8,17 s, 3H ($NH_3^+$); 10,92-10,93 d, 1H (NH); 11,44 s, 1H (NH+).
Point de fusion : 149-151°C.

**Exemple 21 - Chlorhydrate de la 4-(napht-1-yl) 1-(tryptamine 5-O-éthyl) pipérazine :**

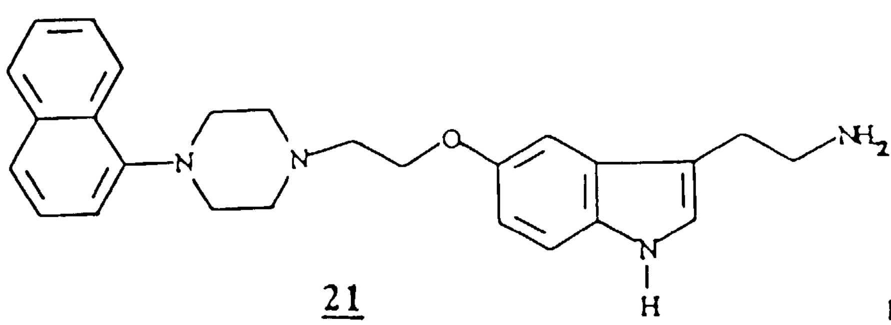

21

HCl

Le composé 21 est obtenu à partir duc omposé 20A (1388 mg; 4,09 mmol) et de 1-naphtyl pipérazine (870 mg; 4.09 mmol) selon la procédure décrite pour la préparation de l'exemple 20. La purification du produit sous forme de base est effectuée par chromatographie sur colonne de gel de silice éluée par un mélange chloroforme/méthanol/ammoniaque (85:14:1; v/v). Le produit pur est isolé sous forme de sirop incolore qui conduit, après traitement à l'acide chlorhydrique dans l'éther, au composé attendu (1064 mg; 72%).

Analyse élémentaire ($C_{26}H_{31}N_4O_1Cl_1$), % calculés : C 69,24; H 6,93; N 12,42; Cl 7,86; % trouvés : C 68,78; H 6,85; N 12,26; Cl 7,25.

Spectre de résonnance magnétique nucléaire du proton, DMSO-$d_6$ (ppm) : 2,85-3,37 m, 14H ($CH_2$); 4,15-4,21 t, 2H ($CH_2$); 6,78-8,16 m, 13H (Ar + $NH_3$+); 10,86 s, 1H (NH).

Point de fusion : 122°C.

**Exemple 22 - Chlorhydrate de la 4-(3,4-dichloro-phényl) 1-(tryptamine 5-O-éthyl) pipérazine :**

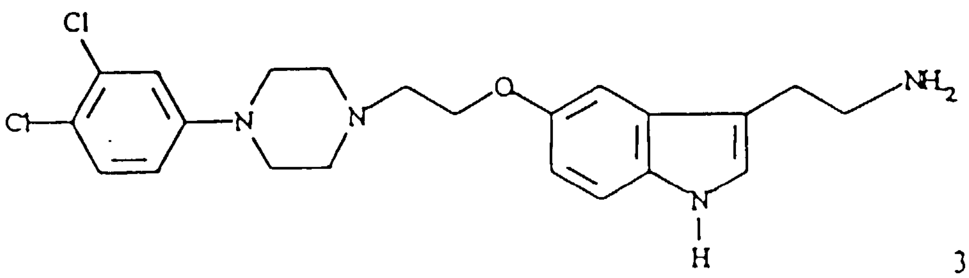

3HCl

22

Le composé 22 est obtenu à partir du composé 20A (1000 mg; 2,95 mmol) et de 3,4-dichloro-phényl pipérazine (682 mg; 2,95 mmol) selon la procédure décrite pour la préparation de l'exemple 20. La purification du produit sous forme de base est effectuée par chromatographie sur colonne de gel de silice éluée par un mélange chloroforme/méthanol/ammoniaque (80:19:1; v/v). Le produit pur est isolé sous forme de sirop beige qui conduit, après traitement à l'acide chlorhydrique dans l'éther, au composé attendu (1,11 g, 75%).

Analyse élémentaire ($C_{22}H_{29}N_4O_1Cl_5$), % calculés : C 48,69; H 5,39; N 10,32; Cl 32,66 % trouvés : C 48,16; H 5,38; N 10,02; Cl 30,82.

Spectre de résonnance magnétique nucléaire du proton, DMSO-$d_6$ (ppm) : 3,00-3,94 m, 14 H ($CH_2$); 4,85 d, 2H ($CH_2$); 6,77-7,47 m, 7H (Ar); 8,19 s, 3H ($NH_3$+); 10,92 s, 1H (NH); 11,62 s, 1H (NH).

Point de fusion : 154°C.

**ETUDES D'AFFINITE POUR LES RECEPTEURS 5-$HT_{1D}$**

Cette étude est réalisée selon la technique décrite par Herrick-Davis et Titeler (J. Neurochem. 50, 1624-1631, 1988).

**- Préparation des membranes :**

Des cerveaux de mouton sont prélevés à l'abattoir local et transportés dans la glace. Les noyaux caudés sont prélevés, pesés et homogénéisés au Polytron pendant 20 sec (vitesse 6-7) dans 20 volumes de Tris-HCl 50 mM, pH 7,7. L'homogénat est centrifugé 10 mn à 48000 g avec une centrifugeuse L5 50E (Beckman).

Le culot repris par 20 volumes de Tris-HCl 50 mM, pH 7,7 est placé dans un bain-marie à 37°C pendant 10 mn, puis recentrifugé 10 mn à 48000 g. Le culot alors obtenu est aussitôt congelé en fractions de 0.5 g de tissu.

**- Affinités :**

Le culot est décongelé et homogénéisé au Dounce dans 80 volumes de Tris-HCl, 50 mM pH 7,7 contenant 4 mM $CaCl_2$, 10 μM de pargyline et 0,1 % d'acide ascorbique.

La liaison est réalisée à 25° C en incubant pendant 30 mn :

- 0,1 ml de tampon ou 10 μM en concentration finale de sumatriptan pour obtenir la liaison non spécifique, ou le produit faisant partie de cette invention à différentes concentrations (entre $10^{-10}$ et $10^{-6}$M).

- 0,8 ml de suspension de membrane
- 0,1 ml de $^3H$-5-HT (15 à 30 Ci/mM, New England Nuclear France)

L'incubation est terminée par la filtration rapide sur filtres GF/B et rinçage avec 3 fois 3 ml de tampon glacé, à l'aide d'un harvester de fabrication Brandel permettant de filtrer 48 échantillons. Les filtres sont introduits dans des fioles contenant 4 ml de liquide scintillant emulsifier-safe (Packard) et la radioactivité mesurée avec un compteur Tri-carb 4640 (Packard).

L'$CI_{50}$ (concentration qui inhibe de 50 % l'affinité spécifique) est déterminée graphiquement.

Les $CI_{50}$ des différents produits faisant partie de cette invention pour les autres récepteurs ont été mesurées selon les techniques décrites dans les références suivantes:

* Récepteurs 5-$HT_{1A}$ et $_B$
  Peroutka S.J. Pharmacological différentiation and characterization of 5-$HT_{1A}$, 5-$HT_{1B}$ and 5-$HT_{1C}$ binding sites in rat frontal cortex. J. Neurochem., 45, 529-540, 1986.

* Récepteur 5-$HT_{1C}$
  Pazos A., Hoyer D. and Palacios J.M. The binding sites of serotonergic ligands to the porcine choroid plexus : characterization of a new type of serotonin récognition site. Eur. J. Pharmacol., 106, 539-546, 1985.

* Récepteur 5-$HT_2$
  Leysen J.E., Niemegeers C.J.E., Van Nueten J.M. and Laduron P.M. $^3H$-ketanserine (R 41468), a selective $^3H$ ligand for serotonin2 receptor binding sites. Mol. Pharmacol., 21, 301-330, 1982.

* Récepteur $\alpha$ 1 - adrénergique
  Leslie Morrow A. and Creese I. Characterization of $\alpha$1-adrenergic receptor subtypes in rat brain : a réévaluation of $^3H$-WB4101 and $^3H$ prazosin binding. Mol. Pharmacol. 29, 321-330, 1986.

* Récepteur $\alpha$ 2 - adrénergique
  Mallard N.J., Tyacke R., Hudson A.L. and Nutt D.S. Comparative binding studies of $^3H$-idazoxan and $^3H$-RX821002 in the rat brain. Brit.J.Pharmacol. 102, 221P, 1991.

* Récepteur D2 - dopaminergique
  Nisnik H.B., Grigoriadis D.E., Pri-Bar I., Buchman O. and Seeman P. Dopamine D2 receptors selectively labeled by a benzamide neuroleptic : $^3H$-YM-09159-2 Naunyn-Schmiedeberg's Arch. Pharmacol. 329, 333-343, 1985.

**Profil d'affinité des molécules (CI50 x 10-9M)**

| Exemple* | α 2 | α 1 | D2 | $5\text{-HT}_{1D}$ | | $5\text{-HT}_{1A}$ | | $5\text{-HT}_{1B}$ | | $5\text{-HT}_{1C}$ | $5\text{-HT}_2$ | Ratio[x] |
|---|---|---|---|---|---|---|---|---|---|---|---|---|
| | | | | moyen. | STD# | moyen. | STD# | moyen. | STD# | | | 1A/1D |
| 1D | 7000 | 8500 | > 100 | 6.5 | 1.5 | 120 | | - | | - | 2000 | 18.5 |
| 3 | > 1000 | > 1000 | > 1000 | 7 | 0.7 | 33.5 | 14.8 | 1.9 | 0.1 | 4000 | > 1000 | 4.8 |
| 1 | > 1000 | > 1000 | > 1000 | 2.1 | 0.1 | 23.5 | 4.9 | 0.6 | | 3000 | > 1000 | 11.2 |
| 4 | > 1000 | > 1000 | > 1000 | 3.2 | 0.2 | 115 | 70.7 | 1.9 | 0.5 | 1800 | > 1000 | 35.9 |
| 5 | > 1000 | > 1000 | > 1000 | 29.3 | 26.6 | 240 | 56.6 | 6 | 0.7 | > 1000 | > 1000 | 8.2 |
| 5A | > 1000 | > 1000 | > 1000 | 2 | 0.3 | 34.5 | 0.7 | 1 | 0.1 | 1000 | > 1000 | 17.3 |
| 6A | > 1000 | > 1000 | > 1000 | 16 | 5.7 | 100 | 14.1 | 1.5 | | > 1000 | > 1000 | 6.3 |
| 7 | > 1000 | > 1000 | > 1000 | 725 | 35.4 | > 1000 | | 355 | 148.5 | > 1000 | > 1000 | > 1.4 |
| 6 | > 1000 | > 1000 | > 1000 | 2.9 | 1.0 | 23.5 | 2.1 | 1.1 | 0.7 | > 1000 | > 1000 | 8.1 |
| 8 | - | - | - | > 1000 | | > 1000 | | 1000 | | - | - | |
| 9 | > 1000 | > 1000 | > 1000 | 190 | 42.4 | 325 | 7.1 | 85 | 21.2 | 600 | 550 | 1.7 |
| 10 | > 1000 | > 1000 | > 1000 | 710 | 14.1 | 465 | 21.2 | 335 | 233.3 | > 1000 | > 1000 | 0.7 |
| 11 | - | - | - | 6.0 | - | 125 | - | 5.5 | - | 1000 | > 1000 | 20.8 |

* Les exemples sont ceux décrits dans le texte pour illustrer l'invention.

\# STD correspond à l'écart type.

[x] Rapport des $CI_{50}$ de chaque produit pour les récepteurs $5HT_{1A}$ et $5HT_{1D}$.

Les nouveaux composés indoliques dérivés de pipérazine faisant partie de cette invention sont des ligands ayant une affinité exceptionnelle pour les récepteurs 5 $HT_{1D}$ et 5 $HT_{1B}$ comme le démontrent les exemples décrits ci-dessus. De nombreux composés, faisant partie intégrante de la présente invention, présentent en outre l'avantage d'être particulièrement sélectifs pour le récepteur 5 $HT_{1D}$ par rapport aux récepteurs 5 $HT_{1A}$, 5 $HT_{1C}$, 5 $HT_2$, $\alpha_1$, $\alpha_2$, $D_2$, La sélectivité des composés de la présente invention et en particulier leur affinité préférentielle pour le récepteur 5 $HT_{1D}$ par rapport au récepteur 5 $HT_{1A}$ représente un avantage très important par rapport aux ligands du récepteur 5 $HT_{1D}$ connus jusqu'ici (cf. Annual Reports in Medicinal Chemistry, vol 27, chapitre 3, p.25 ; Academic Press, 1992).

En thérapeutique humaine, les composés de formule générale (I) selon l'invention sont particulièrement utiles pour le traitement et la prévention des désordres liés à la sérotonine. Ces composés peuvent donc être utilisés dans le traitement et la prévention de la dépression, des désordres compulsifs obsessionnels, des désordres alimentaires et en particulier de la boulimie, de l'agressivité, de l'alcoolisme, du tabagisme, de l'hypertension, de la nausée, du dysfonctionnement sexuel, du comportement asocial, de l'anxiété, de la migraine, de la maladie d'Alzheimer et des troubles de la mémoire.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale, topique ou tout autre voie d'administration.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée ; elles sont généralement comprises entre 0,001 g et 1 g (de préférence comprises entre 0,005 g et 0,25 g) par jour de préférence par voie orale pour un adulte avec des doses unitaires allant de 0,1 mg à 500 mg de substance active, de préférence de 1 mg à 50 mg.

D'une façon générale, le médecin déterminera la posologie appropriée en fonction de l'age, du poids et de tous les autres facteurs propres au sujet à traiter. Les exemples suivants illustrent des compositions selon l'invention [dans ces exemples, le terme "composant actif" désigne un ou plusieurs (généralement un) des composés de formule (I) selon la présente invention] :

Comprimés

On peut les préparer par compression directe ou en passant par une granulation au mouillé. Le mode opératoire par compression directe est préféré mais il peut ne pas convenir dans tous les cas selon les doses et les propriétés physiques du composant actif.

| *A - Par compression directe* | |
|---|---|
| | mg pour 1 |
| comprimé | |
| composant actif | 10,0 |
| cellulose microcristalline B.P.C. | 89,5 |
| stéarate de magnésium | 0,5 |
| | 100,0 |

On passe le composant actif au travers d'un tamis à ouverture de maille de 250 µm de côté, on mélange avec les excipients et on comprime à l'aide de poinçons de 6,0 mm. On peut préparer des comprimés présentant d'autres résistances mécaniques en modifiant le poids de compression avec utilisation de poinçons appropriés.

| *B - Granulation au mouillé* | |
|---|---|
| | mg pour un comprimé |
| composant actif | 10,0 |
| lactose Codex | 74,5 |
| amidon Codex | 10,0 |
| amidon de maïs prégélatinisé Codex | 5,0 |
| stéarate de magnésium | 0,5 |
| Poids à la compression | 100,0 |

On fait passer le composant actif au travers d'un tamis à ouverture de maille de 250 µm et on mélange avec le lactose, l'amidon et l'amidon prégélatinisé. On humidifie les poudres mélangées par de l'eau purifiée, on met à l'état de granulés, on sèche, on tamise et on mélange avec le stéarate de magnésium. Les granulés lubrifiés sont mis en comprimés comme pour les formules par compression directe. On peut appliquer sur les comprimés une pellicule de revêtement au moyen de matières filmogènes appropriées, par exemple la méthylcellulose ou l'hydroxy-propyl-méthyl-cellulose, selon des techniques classiques. On peut également revêtir les comprimés de sucre.

| Capsules | |
|---|---|
| | mg pour une capsule |
| composant actif | 10,0 |
| * amidon 1500 | 89,5 |
| stéarate de magnésium Codex | 0,5 |
| Poids de remplissage | 100,0 |

*une forme d'amidon directement compressible provenant de la firme Colorcon Ltd, Orpington, Kent, Royaume Uni.

On fait passer le composant actif au travers d'un tamis à ouverture de maille de 250 µm et on mélange avec les autres substances. On introduit le mélange dans des capsules de gélatine dure n°2 sur une machine à remplir appropriée. On peut préparer d'autres unités de dosage en modifiant le poids de remplissage et, lorsque c'est nécessaire, en changeant la dimension de la capsule.

**Sirop**

| | mg par dose de 5 ml |
|---|---|
| composant actif | 10,0 |

| | | | |
|---|---|---|---|
| saccharose Codex | | | 2750,0 |
| glycérine Codex | | | 500,0 |
| tampon | ) | | |
| arôme | ) | | |
| colorant | ) | | q.s. |
| préservateur | ) | | |
| eau distillée | | | 5,0 |

On dissout le composant actif, le tampon, l'arôme, le colorant et le préservateur dans une partie de l'eau et on ajoute la glycérine. On chauffe le restant de l'eau à 80°C et on y dissout le saccharose puis on refroidit. On combine les deux solutions, on règle le volume et on mélange. Le sirop obtenu est clarifié par filtration.

| Suppositoires | |
|---|---|
| Composant actif | 10,0 mg |
| * Witepsol H15 complément à | 1,0 g |

* Marque commercialisée pour Adeps Solidus de la Pharmacopée Européenne.

On prépare une suspension du composant actif dans le Witepsol H15 et on l'introduit dans une machine appropriée avec moules à suppositoires de 1 g.

| Liquide pour administration par injection intraveineuse | |
|---|---|
| | g/l |
| composant actif | 2,0 |
| eau pour injection Codex complément à | 1000,0 |

On peut ajouter du chlorure de sodium pour régler la tonicité de la solution et régler le pH à la stabilité maximale ét/ou pour faciliter la dissolution du composant actif au moyen d'un acide ou d'un alcali dilué ou en ajoutant des sels tampons appropriés. On prépare la solution, on la clarifie et on l'introduit dans des ampoules de dimension appropriée qu'on scelle par fusion du verre. On peut également stériliser le liquide pour injection par chauffage à l'autoclave selon l'un des cycles acceptables. On peut également stériliser la solution par filtration et introduire en ampoule stérile dans des conditions aseptiques. La solution peut être introduite dans les ampoules en atmosphère gazeuse.

| Cartouches pour inhalation | |
|---|---|
| | g/cartouche |
| composant actif mironisé | 1,0 |
| lactose Codex | 39,0 |

Le composant actif est micronisé dans un broyeur à énergie de fluide et mis à l'état de fines particules avant mélange avec du lactose pour comprimés dans un mélangeur à haute énergie. Le mélange pulvérulent est introduit en capsules de gélatine dure n°3 sur une machine à encapsuler appropriée. Le contenu des cartouches est administré à l'aide d'un inhalateur à poudre.

| Aérosol sous pression à valve doseuse | | |
|---|---|---|
| | mg/dose | pour 1 boîte |
| composant actif micronisé | 0,500 | 120 mg |
| acide oléique Codex | 0,050 | 12 mg |
| trichloroofluorométhane pour usage pharmaceutique | 22,25 | 5,34 g |
| dichlorodifluorométhane pour usage pharmaceutique | 60,90 | 14,62 g |

Le composant actif est micronisé dans un broyeur à énergie de fluide et mis à l'état de fines particules. On mélange l'acide oléique avec le trochlorofluorométhane à une température de 10-15°C et on introduit dans la solution à l'aide d'un mélangeur à haut effet de cisaillement le médicament micronisé. La suspension est introduite en quantité mesurée dans des boîtes aérosol en aluminium sur lesquelles on fixe des valves doseuses appropriées délivrant une dose de 85 mg de la suspension ; le dichlorodifluorométhane est introduit dans les boîtes par injection au travers des valves.

## Revendications

1. Composé répondant à la formule générale (I) :

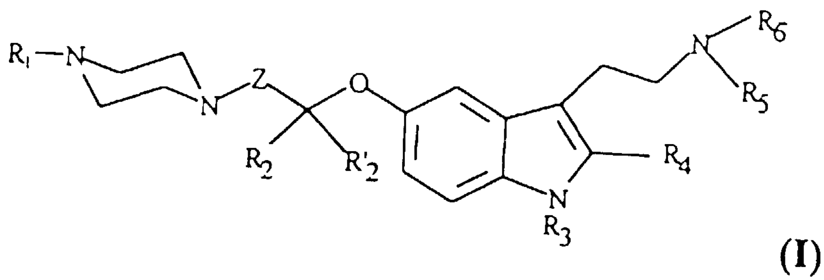

(I)

dans laquelle

$R_1$ représente un atome d'hydrogène, un radical alkyle ramifié ou linéaire en $C_1$ à $C_6$ ou un radical phényle, benzyle, cycloalkyle en $C_3$ à $C_7$, polycycloalkyle en $C_7$ à $C_{12}$, dibenzocycloalkyle, dibenzooxépine, dibenzoazépine, dibenzothiépine, benzopyrrolocycloalkyle, benzothiénocycloalkyle, naphtyle, éventuellement substitués par un ou plusieurs substituants choisis parmi les atome d'halogène et-les radicaux alkyle en $C_1$ à $C_6$, alcoxy et alkylthio dont les fragments alkyle sont en $C_1$ à $C_6$,
Z représente C=O, $SO_2$, ou $(CH_2)_n$ dans lequel n est compris entre 1 et 5,
$R_2$ représente un atome d'hydrogène, un radical alkyle ramifié ou linéaire en $C_1$ à $C_6$, ou, un radical phényle, benzyle, cycloalkyle, pyrrole, furane, pyridinyle, thiophényle, éventuellement substitués par un ou plusieurs substituants choisis parmi les atomes d'halogène et les radicaux alkyle, alcoxy et alkylthio dont les fragments alkyle sont en $C_1$ à $C_6$,
$R'_2$ représente un atome d'hydrogène, un radical alkyle ramifié ou linéaire en $C_1$ à $C_6$ ou un radical phényle.
$R_3$ représente un atome d'hydrogène, un radical alkyle ramifié ou linéaire en $C_1$ à $C_6$, ou un radical benzyle ou phénéthyle,
$R_4$ représente un atome d'hydrogène, de chlore, de fluor ou de brome ou un radical alkyle linéaire ou ramifié en $C_1$ à $C_6$,
$R_5$ représente un atome d'hydrogène, un radical alkyle ramifié ou linéaire en $C_1$ à $C_6$ ou un radical benzyle ou phénéthyle,
$R_6$ représente un atome d'hydrogène, un radical alkyle linéaire ou ramifié en $C_1$ à $C_6$, un radical acycle ($COR_7$), acyloxy ($CO_2R_7$) ou acylamino ($CONHR_7$) dans lesquels $R_7$ représente un radical alkyle linéaire ou ramifié en $C_1$ à $C_6$, ou un radical phényle,

leurs sels et solvats acceptables pour usage thérapeutique.

2. Composés selon la revendication 1, caractérisés en ce que $R_1$ représente un groupe phényle ou un naphtyle éventuellement substitué par un ou plusieurs substituants choisis parmi les atomes d'halogène et les radicaux alkyle en $C_1$ à $C_6$, alcoxy et alkylthio dont les fragments alkyle sont en $C_1$ à $C_6$.

3. Composés selon la revendication 1, caractérisés en ce que $R_1$ représente une dibenzothiépine.

4. Composés selon la revendication 1, caractérisés en ce que Z représente CO.

5. Composés selon la revendication 1, caractérisés en ce que Z représente $SO_2$.

6. Composés selon la revendication 1, caractérisés en ce que Z représente $(CH_2)_n$ dans lequel n est compris entre

1 et 5.

**7.** Composés selon la revendication 1, caractérisés en ce que $R_2$ et $R'_2$ représentent un hydrogène.

**8.** Composés selon la revendication 1, caractérisés en ce que $R_2$ représente un hydrogène.

**9.** Composés selon la revendication 1, caractérisés en ce que $R_2$ représente un méthyle.

**10.** Composés selon la revendication 1, caractérisés en ce que $R_3$ représente un hydrogène.

**11.** Composés selon la revendication 1, caractérisés en ce que $R_4$ représente un hydrogène.

**12.** Composés selon la revendication 1, caractérisés en ce que $R_5$ et $R_6$ représentent un hydrogène.

**13.** Composés selon la revendication 1, caractérisés en ce que $R_5$ est un radical alkyle, linéaire ou ramifié en $C_1$ à $C_6$.

**14.** Composés selon la revendication 1, caractérisés en ce que $R_5$ et $R_6$ représentent un radical alkyle, linéaire ou ramifié, en $C_1$ à $C_6$.

**15.** Composés selon l'une des revendications 1 à 14, à l'état de sel acceptable pour l'usage thérapeutique caractérisés en ce que ces sels sont des chlorhydrates, des bromhydrates, des sulfates, des fumarates ou des maléates.

**16.** Un composé choisi parmi :

1) Chlorhydrate de la 4-*o*-tolyl 1-(tryptamine 5-O-carboxyméthyl) pipérazide
2) Chlorhydrate de la 4-(α, α, α-trifluoro-m-tolyl) 1-(tryptamine 5-O-carboxyméthyl) pipérazide
3) Chlorhydrate de la 4-(napht-1-yl) 1-(tryptamine 5-O-carboxyméthyl) pipérazide
4) Chlorhydrate de la 4-(2,3-xylyl) 1-(tryptamine 5-O-carboxyméthyl) pipérazide
5) Chlorhydrate de la 4-(m-méthoxyphényl) 1-(tryptamine 5-O-carboxyméthyl) pipérazide
6) Chlorhydrate de la 4-méthyl 1-(tryptamine 5-O-carboxyméthyl) pipérazide
7) Chlorhydrate de la 1-[(terbutoxycarbamate)-tryptamine 5-O-carboxyméthyl]-4-[10-(8-méthylthio-10-11-dihydrodibenzo [b,f]thiépine)] pipérazide
8) Chlorhydrate de la 4-*O*-tolyl 1-[tryptamine 5-O (α-phényl-carboxyméthyl)] pipérazide
9) Chlorhydrate de la 4-naphtyl 1-[tryptamine 5-O (α-phényl-carboxyméthyl)] pipérazide
10) Chlorhydrate de la 4-o-tolyl 1-[tryptamine 5-O (α-méthyl-carboxyméthyl)] pipérazide
11) Chlorhydrate de la 4-(napht-1-yl) 1-(tryptamine 5-O-méthylsulfonyl) pipérazide
12) Chlorhydrate de la 4-*m*-chlorophényl 1-(tryptamine 5-O propyl) pipérazine
13) Chlorhydrate de la 4-(4-acétyl-phényl) 1-(tryptamine 5-O-carboxyméthyl) pipérazide
14) Chlorhydrate de la 4-(2,4-diméthyl-phényl) 1-(tryptamine 5-O-carboxyméthyl) pipérazide
15) Chlorhydrate de la 4-(4-chloro-phényl) 1(tryptamine 5-O-carboxyméthyl) pipérazide
16) Chlorhydrate de la 4-(3,4-dichloro-phényl) 1-(tryptamine 5-O-carboxyméthyl) pipérazide
17) Chlorhydrate de la 4-(4-méthoxy-phényl) 1-(tryptamine 5-O-carboxyméthyl) pipérazide
18) Chlorhydrate de la 4-cyclopentyl 1-(tryptamine 5-O-carboxyméthyl) pipérazide
19) Chlorhydrate de la 4-(o-tolyl) 1-(tryptamine 5-O-éthyl) pipérazine
20) Chlorhydrate de la 4-(napht-1-yl) 1-(tryptamine 5-O-éthyl) pipérazine
21) Chlorhydrate de la 4-(3,4-dichloro-phényl) 1-(tryptamine 5-O-éthyl) pipérazine

**17.** Procédé de préparation des composés de formule générale (I) selon la revendication 1 ou de leurs sels ou solvats acceptables pour l'usage thérapeutique, caractérisé en ce que l'on fait réagir un composé de formule générale (II) :

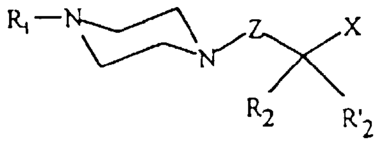

(II)

dans laquelle $R_1$, Z, $R_2$ et $R'_2$ ont les significations indiquées en référence à la formule générale (I) et où X est défini comme un groupe partant tel qu'un halogène (brome, iode ou chlore), un mésylate, un tosylate ou un triflate, avec un dérivé de la sérotonine de formule générale (III) :

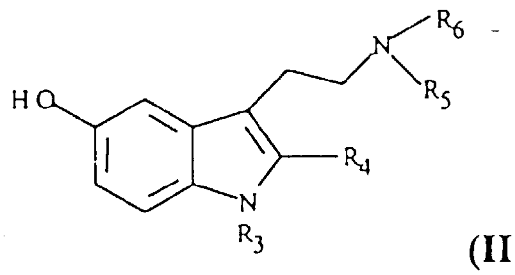

(III)

dans laquelle $R_3$, $R_4$, $R_5$ et $R_6$ ont la signification indiquée en référence à la formule générale (I), et $R_6$ est différent d'un atome d'hydrogène.

**18.** Procédé de préparation des composés de formule (I) selon la revendication 1, dans laquelle Z représente CO, caractérisé en ce que l'on fait réagir un composé de formule générale (V) :

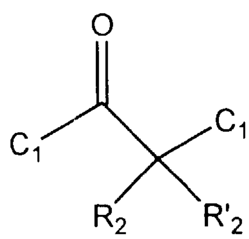

(V)

dans laquelle $R_2$ et $R'_2$ ont la signification indiquée en référence à la formule générale (I) avec, tout d'abord, un dérivé de formule générale (IV) :

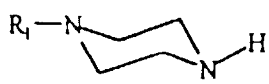

(IV)

dans laquelle $R_1$ est défini comme dans la formule générale (I), puis avec un dérivé de formule générale (III) selon la revendication 17, dans laquelle $R_3$, $R_4$, $R_5$ et $R_6$ sont définis comme dans la formule générale (I).

**19.** Procédé selon l'une des revendications 17 et 18 caractérisé en ce que l'on convertit un composé de formule générale (I) ou un sel ou dérivé protégé d'un tel composé en un autre composé de formule générale (I), et, ou,

l'on convertit un composé de formule générale (I) ou un sel d'un tel composé en un sel ou solvat acceptable pour l'usage pharmaceutique.

**20.** Procédé de préparation des composés de formule générale (I) selon la revendication I caractérisé en ce que l'on fait réagir un dérivé de formule (IV) selon la revendication 18, dans laquelle $R_1$ a la signification indiquée en référence à la formule (I) avec un dérivé de formule (VII) :

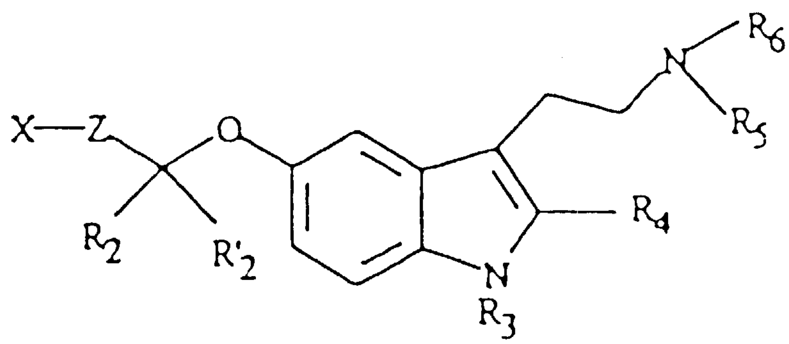

(VII)

dans laquelle Z, $R_2$, $R'_2$, $R_3$, $R_4$ et $R_6$ ont la signification indiquée en référence à la formule (I) et $R_6$ est différent d'un atome d'hydrogène, et dans laquelle X représente un groupe partant tel qu'un halogène, un mésylate, un tosylate, un triflate ou le groupe X-Z représente un ester activé propice à la formation d'une amide.

**21.** Compositions pharmaceutiques contenant, à titre d'ingrédients actifs, un composé selon l'une des revendications 1 à 16 en combinaison avec un véhicule pharmaceutique acceptable, pour le traitement tant curatif que préventif des désordres liés à la sérotonine.

**22.** Compositions pharmaceutiques contenant, à titre d'ingrédients actifs, un composé selon l'une des revendications 1 à 16 en combinaison avec un véhicule pharmaceutique acceptable, pour le traitement de la dépression.

**23.** Compositions pharmaceutiques contenant, à titre d'ingrédients actifs, un composé selon l'une des revendications 1 à 16 en combinaison avec un véhicule pharmaceutique acceptable, pour le traitement des désordres compulsifs obsessionnels.

**24.** Compositions pharmaceutiques contenant, à titre d'ingrédients actifs, un composé selon l'une des revendications 1 à 16 en combinaison avec un véhicule pharmaceutique acceptable, pour le traitement de l'anxiété.

**25.** Compositions pharmaceutiques contenant, à titre d'ingrédients actifs, un composé selon l'une des revendications 1 à 16 en combinaison avec un véhicule pharmaceutique acceptable, pour le traitement de l'agressivité et/ou de l'alcoolisme et/ou du comportement asocial.

**26.** Compositions pharmaceutiques contenant, à titre d'ingrédients actifs, un composé selon l'une des revendications 1 à 16 en combinaison avec un véhicule pharmaceutique acceptable, pour le traitement de la migraine.

**27.** Compositions pharmaceutiques contenant, à titre d'ingrédients actifs, un composé selon l'une des revendications 1 à 16 en combinaison avec un véhicule pharmaceutique acceptable, pour le traitement des désordres alimentaires, en particulier la boulimie.

**28.** Compositions pharmaceutiques caractérisées en ce qu'elles contiennent en tant que substances actives au moins un composé selon l'une quelconque des revendications 1 à 16 en combinaison avec un véhicule pharmaceutique acceptable pour le traitement des dysfonctionnements sexuels.

**Patentansprüche**

1. Der allgemeinen Formel (I) entsprechende Verbindung:

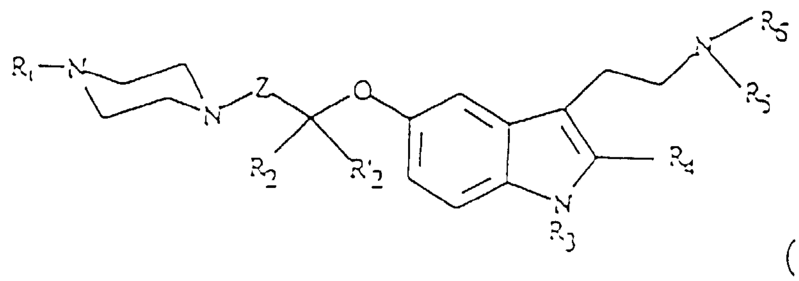

(I)

wobei

$R_1$ ein Wasserstoffatom, ein verzweigtes oder lineares $C_1$ bis $C_6$ Alkylradikal oder ein Phenyl-, Benzyl, $C_3$ bis $C_7$ Cycloalkyl-, $C_7$ bis $C_{12}$ Polycycloalkyl-, Dibenzocycloalkyl-, Dibenzooxepin-, Dibenzoazepin-, Dibenzothiepin-, Benzopyrrolocycloalkyl-, Benzothienocycloalkyl-, Naphthylradikal darstellt, gegebenenfalls substituiert durch einen oder mehrere Substituenten, die ausgewählt werden aus Halogenatomen und $C_1$ bis $C_6$ Alkyl-, Alkoxy- und Alkylthioradikalen, deren Alkylreste $C_1$ bis $C_6$ aufweisen, Z C=0, $SO_2$ oder $(CH_2)_n$ darstellt, wobei n zwischen 1 und 5 liegt, $R_2$ ein Wasserstoffatom, ein verzweigtes oder lineares $C_1$ bis $C_6$ Alkylradikal oder ein Phenyl-, Benzyl-, Cycloalkyl-, Pyrrol-, Furan-, Pyridinyl-, Thiophenylradikal darstellt, gegebenenfalls substituiert durch einen oder mehrere Substituenten die ausgewählt werden unter den Halogenatomen und den Alkyl-, Alkoxy- und Alkylthioradikalen, deren Alkylreste $C_1$ bis $C_6$ aufweisen,
$R'_2$ ein Wasserstoffatom, ein verzweigtes oder lineares $C_1$ bis $C_6$ Alkylradikal oder ein Phenylradikal darstellt,
$R_3$ ein Wasserstoffatom, ein verzweigtes oder lineares $C_1$ bis $C_6$ Alkylradikal oder ein Benzyl- oder Phenethylradikal darstellt,
$R_4$ ein Wasserstoff-, Chlor-, Fluor- oder Bromatom oder ein lineares oder verzweigtes $C_1$ bis $C_6$ Alkylradikal darstellt,
$R_5$ ein Wasserstoffatom, ein verzweigtes oder geradkettiges $C_1$ bis $C_6$ Alkylradikal oder ein Benzyl- oder Phenetylradikal darstellt,
$R_6$ ein Wasserstoffatom, ein lineares oder verzweigtes $C_1$ bis $C_6$ Alkylradikal, ein Acyl- ($COR_7$), Acyloxy- ($CO_2R_7$) oder Acylaminoradikal ($CONHR_7$) darstellt, wobei $R_7$ ein lineares oder verzweigtes $C_1$ bis $C_6$ Alkylradikal oder ein Phenylradikal darstellt,

ihre für die therapeutische Verwendung verträglichen Salze und Solvate.

2. Verbindungen nach Anspruch 1, dadurch charakterisiert, daß $R_1$ eine Phenyl- oder Naphthylgruppe darstellt, gegebenenfalls substituiert durch einen oder mehrere Substituenten, die ausgewählt werden unter den Halogenatomen und den $C_1$ bis $C_6$ Alkyl-, Alkoxy- und Alkylthioradikalen, deren Alkylreste $C_1$ bis $C_6$ aufweisen.

3. Verbindungen nach Anspruch 1, dadurch charakterisiert, daß $R_1$ Dibenzothiepin darstellt.

4. Verbindungen nach Anspruch 1, dadurch charakterisiert, daß Z CO darstellt.

5. Verbindungen nach Anspruch 1, dadurch charakterisiert, daß Z $SO_2$ darstellt.

6. Verfahren nach Anspruch 1, dadurch charakterisiert, daß Z $(CH_2)_n$ darstellt, wobei n zwischen 1 und 5 liegt.

7. Verbindungen nach Anspruch 1, dadurch charakterisiert, daß $R_2$ und $R'_2$ Wasserstoff darstellen.

8. Verbindungen nach Anspruch 1, dadurch charakterisiert, daß $R_2$ Wasserstoff darstellt.

9. Verbindungen nach Anspruch 1, dadurch charakterisiert, daß $R_2$ Methyl darstellt.

10. Verbindungen nach Anspruch 1, dadurch charakterisiert, daß $R_3$ Wasserstoff darstellt.

**11.** Verbindungen nach Anspruch 1, dadurch charakterisiert, daß $R_4$ Wasserstoff darstellt.

**12.** Verbindungen nach Anspruch 1, dadurch charakterisiert, daß $R_5$ und $R_6$ Wasserstoff darstellen.

**13.** Verbindungen nach Anspruch 1, dadurch charakterisiert, daß $R_5$ ein lineares oder verzweigtes $C_1$ bis $C_6$ Alkylradikal ist.

**14.** Verbindungen nach Anspruch 1, dadurch charakterisiert, daß $R_5$ und $R_6$ ein lineares oder verzweigtes $C_1$ bis $C_6$ Alkylradikal darstellen.

**15.** Verbindungen nach einem der Ansprüche 1 bis 14 als verträgliches Salz zur therapeutischen Verwendung, dadurch charakterisiert, daß die Salze Hydrochloride, Hydrobromide, Sulfate, Fumarate oder Maleate sind.

**16.** Verbindung, ausgewählt unter:

1) Hydrochlorid von 4-o-Tolyl-1-(tryptamin-5-O-carboxymethyl)piperazid
2) Hydrochlorid von 4-(α,α,α-Trifluoro-m-tolyl-1-(tryptamin-5-O-carboxymethyl)piperazid
3) Hydrochlorid von 4-(Naphth-1-yl)-1-(tryptamin-5-O-carboxymethyl)piperazid
4) Hydrochlorid von 4-(2,3-Xylyl)-1-(tryptamin-5-O-carboxymethyl)piperazid
5) Hydrochlorid von 4-(m-Methoxyphenyl-1-(tryptamin-5-O-carboxymethyl)piperazid
6) Hydrochlorid von 4-Methyl-1-(tryptamin-5-O-carboxymethyl)piperazid
7) Hydrochlorid von 1-[(Terbutoxycarbamat)-tryptamin-5-O-carboxymethyl]-4-[10-(8-methylthio-10-11-dihydrobenzo[b,f]thiepin]piperazid
8) Hydrochlorid von 4-o-Tolyl-1-(tryptamin-5-O-α-phenyl-carboxymethyl)piperazid
9) Hydrochlorid von 4-Naphthyl-1-(tryptamin-5-O-(α-phenyl-carboxymethyl)piperazid
10) Hydrochlorid von 4-o-Tolyl-1-(tryptamin-5-O-(α-methyl-carboxymethyl)piperazid
11) Hydrochlorid von 4-(Napht-1-yl)1-(tryptamin-5-O-methylsulfonyl)piperazid
12) Hydrochlorid von 4-m-Chlorophenyl-1-(tryptamin-5-O-propyl)piperazin
13) Hydrochlorid von 4-(4-Acetyl-phenyl)-1-(tryptamin-5-O-carboxymethyl)piperazid
14) Hydrochlorid von 4-(2,4-Dimethyl-phenyl)-1-(tryptamin-5-O-carboxymethyl)piperazid
15) Hydrochlorid von 4-(4-Chloro-phenyl)-1-(tryptamin-5-O-carboxymethyl)piperazid
16) Hydrochlorid von 4-(3,4-Dichloro-phenyl-1-(tryptamin-5-O-carboxymethyl)piperazid
17) Hydrochlorid von 4-(4-Methoxy-phenyl)-1-(tryptamin-5-O-carboxymethyl)piperazid
18) Hydrochlorid von 4-Cyclopentyl-1-(tryptamin-5-O-carboxymethyl)piperazid
19) Hydrochlorid von 4-(o-Tolyl)-1-(tryptamin-5-O-ethyl)piperazid
20) Hydrochlorid von 4-(Napht-1-yl)-1-(tryptamin-5-O-ethyl)piperazid
21) Hydrochlorid von 4-(3,4-Dichloro-phenyl)1-(tryptamin-5-O-ethyl)piperazid.

**17.** Verfahren zur Herstellung von Verbindungen der allgemeinen Formel (I) nach Anspruch 1 oder ihre für die therapeutische Verwendung verträglichen Salze oder Solvate, dadurch charakterisiert, daß man eine Verbindung der allgemeinen Formel (II):

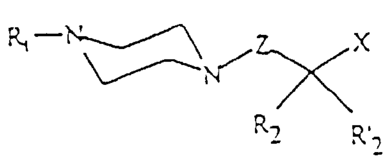

(II)

wobei $R_1$, Z, $R_2$ und $R'_2$ die unter Bezug auf die allgemeine Formel (I) angegebenen Bedeutungen haben und X als Abgangsgruppe wie Halogen (Brom, Iod oder Chlor), Mesylat, Tosylat oder Triflat definiert ist, mit einem Serotoninderivat der allgemeinen Formel (III) reagieren läßt:

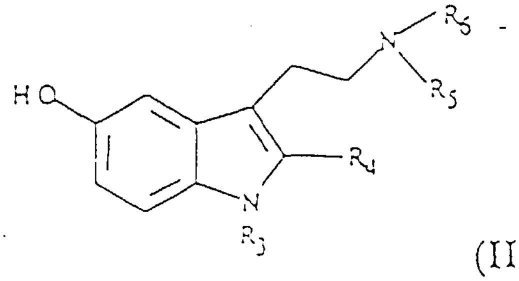

(III)

wobei $R_3$, $R_4$, $R_5$ und $R_6$ die unter Bezug auf die allgemeine Formel (I) angegebenen Bedeutungen haben und $R_6$ von einem Wasserstoffatom verschieden ist.

**18.** Verfahren zur Herstellung von Verbindungen der Formel (I) nach Anspruch 1, wobei Z CO darstellt, dadurch charakterisiert, daß man eine Verbindung der allgemeinen Formel (V):

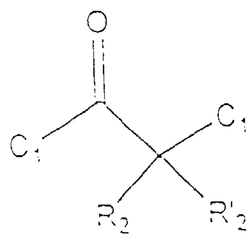

(V)

wobei $R_2$ und $R'_2$ die unter Bezug auf die allgemeine Formel (I) angegebenen Bedeutungen haben, zunächst mit einem Derivat der allgemeinen Formel (IV) reagieren läßt:

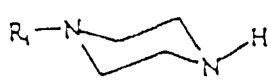

(IV)

wobei $R_1$ wie in der allgemeinen Formel (I) definiert ist, und anschließend mit einem Derivat der allgemeinen Formel (III) nach Anspruch 17, wobei $R_3$, $R_4$, $R_5$ und $R_6$ wie in der allgemeinen Formel (I) definiert sind.

**19.** Verfahren nach einem der Ansprüche 17 und 18, dadurch charakterisiert, daß man eine Verbindung der allgemeinen Formel (I) oder ein Salz oder geschütztes Derivat einer solchen Verbindung in eine andere Verbindung der allgemeinen Formel (I) umwandelt, und daß man eine Verbindung der allgemeinen Formel (I) oder ein Salz einer solchen Verbindung in ein für die pharmazeutische Verwendung geeignetes Salz oder Solvat umwandelt.

**20.** Verfahren zur Herstellung von Verbindungen der allgemeinen Formel (I) nach Anspruch 1, dadurch charakterisiert, daß man ein Derivat der Formel (IV) nach Anspruch 18, wobei $R_1$ die unter Bezug auf die Formel (I) angegebene Bedeutung hat, mit einem Derivat der Formel (VII) reagieren läßt:

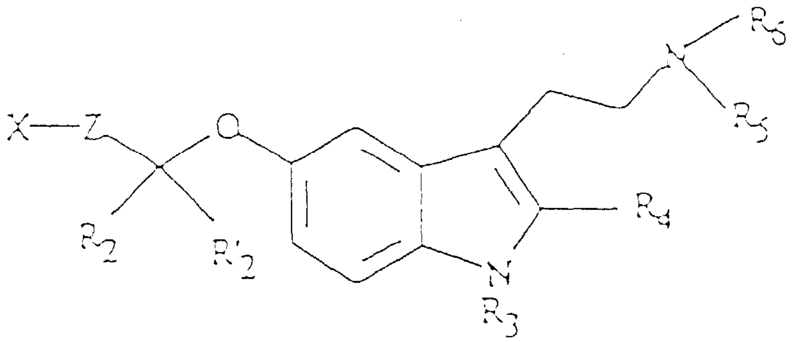

(VII)

wobei Z, $R_2$, $R'_2$, $R_3$, $R_4$ und $R_6$ die unter Bezug auf die Formel (I) angegebene Bedeutung aufweisen und $R_6$ von einem Wasserstoffatom verschieden ist, und wobei X eine Abgangsgruppe darstellt wie ein Halogen, ein Mesylat, ein Tosylat, ein Triflat oder die Gruppe X-Z einen zur Bildung einer Amidgruppe geeigneten aktivierten Ester darstellt.

**21.** Pharmazeutische Zusammensetzung, enthaltend als aktive Bestandteile eine Verbindung nach einem der Ansprüche 1 bis 16 in Kombination mit einem pharmazeutisch verträglichen Vehikel für die kurative oder präventive Behandlung von mit Serotonin zusammenhängenden Erkrankungen.

**22.** Pharmazeutische Zusammensetzung, enthaltend als aktive Bestandteile eine Verbindung nach einem der Ansprüche 1 bis 16 in Kombination mit einem pharmazeutisch verträglichen Vehikel für die Behandlung von Depressionen.

**23.** Pharmazeutische Zusammensetzung, enthaltend als aktive Bestandteile eine Verbindung nach einem der Ansprüche 1 bis 16 in Kombination mit einem pharmazeutisch verträglichen Vehikel für die Behandlung von Zwangserkrankungen.

**24.** Pharmazeutische Zusammensetzung, enthaltend als aktive Bestandteile eine Verbindung nach einem der Ansprüche 1 bis 16 in Kombination mit einem pharmazeutisch verträglichen Vehikel für die Behandlung von Angstzuständen.

**25.** Pharmazeutische Zusammensetzung, enthaltend als aktive Bestandteile eine Verbindung nach einem der Ansprüche 1 bis 16 in Kombination mit einem pharmazeutisch verträglichen Vehikel für die Behandlung von Aggressivität und/oder Alkoholismus und/oder asozialem Verhalten.

**26.** Pharmazeutische Zusammensetzung, enthaltend als aktive Bestandteile eine Verbindung nach einem der Ansprüche 1 bis 16 in Kombination mit einem pharmazeutisch verträglichen Vehikel für die Behandlung von Migräne.

**27.** Pharmazeutische Zusammensetzung, enthaltend als aktive Bestandteile eine Verbindung nach einem der Ansprüche 1 bis 16 in Kombination mit einem pharmazeutisch verträglichen Vehikel für die Behandlung von Ernährungsstörungen, insbesondere von Bulimie.

**28.** Pharmazeutische Zusammensetzungen, dadurch charakterisiert, daß sie als aktive Substanzen mindestens eine Verbindung nach einem der Ansprüche 1 bis 16 in Kombination mit einem pharmazeutisch verträglichen Vehikel enthalten, für die Behandlung von sexuellen Dysfunktionen.

## Claims

**1.** Compounds corresponding to the general formula (I):

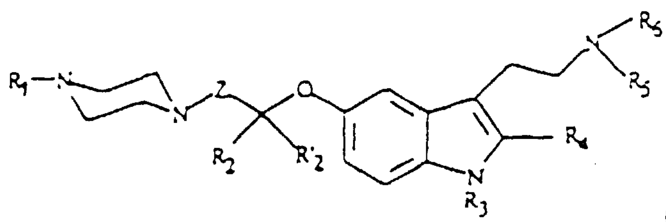

(I)

in which

$R_1$ represents a hydrogen atom, a linear or branched $C_1$ to $C_6$ alkyl radical or a phenyl, benzyl, $C_3$ to $C_7$ cycloalkyl, $C_7$ to $C_{12}$ polycycloalkyl, dibenzocycloalkyl, dibenzooxepine, dibenzoazepine, dibenzothiepine, benzopyrrolocycloalkyl, benzothienocycloalkyl or naphthyl radical, optionally substituted by one or a number of substituents chosen from halogen atoms and alkyl, alkoxy and alkylthio radicals, in which the alkyl fragments are $C_1$ to $C_6$,
Z represents C=O, $SO_2$ or $(CH_2)_n$ in which n is between 1 and 5,
$R_2$ represents a hydrogen atom, a linear or branched $C_1$ to $C_6$ alkyl radical or a phenyl, benzyl, cycloalkyl, pyrrole, furan, pyridinyl or thioophenyl radical, optionally substituted by one or a number of substituents chosen from halogen atoms and alkyl, alkoxy and alkylthio radicals, in which the alkyl fragments are $C_1$ to $C_6$,
$R'_2$ represents a hydrogen atom, a linear or branched $C_1$ to $C_6$ alkyl radical or a phenyl radical,
$R_3$ represents a hydrogen atom, a linear or branched $C_1$ to $C_6$ alkyl radical or a benzyl or phenethyl radical,
$R_4$ represents a hydrogen, chlorine, fluorine or bromine atom or a linear or branched $C_1$ to $C_6$ alkyl radical,
$R_5$ represents a hydrogen atom, a linear or branched $C_1$ to $C_6$ alkyl radical or a benzyl or phenethyl radical,
$R_6$ represents a hydrogen atom, a linear or branched $C_1$ to $C_6$ akyl radical, an acyl ($COR_7$), acyloxy ($CO_2R_7$) or acylamino ($CONHR_7$) radical in which $R_7$ represents a linear or branched $C_1$ to $C_6$ alkyl radical, or a phenyl radical, their salts and solvates which are acceptable in therapeutic use.

**2.** Compounds according to Claim 1, characterized in that $R_1$ represents a phenyl group or a naphthyl optionally substituted by one or a number of substituents chosen from halogen atoms and alkyl, alkoxy and alkylthio radicals, in which the alkyl fragments are $C_1$ to $C_6$.

**3.** Compounds according to Claim 1, characterized in that $R_1$ represents a dibenzothiepine.

**4.** Compounds according to Claim 1, characterized in that Z represents CO.

**5.** Compounds according to Claim 1, characterized in that Z represents $SO_2$.

**6.** Compounds according to Claim 1, characterized in that Z represents $(CH_2)_n$ in which n is between 1 and 5.

**7.** Compounds according to Claim 1, characterized in that $R_2$ and $R'_2$ represent a hydrogen.

**8.** Compounds according to Claim 1, characterized in that $R_2$ represents a hydrogen.

**9.** Compounds according to Claim 1, characterized in that $R_2$ represents a methyl.

**10.** Compounds according to Claim 1, characterized in that $R_3$ represents a hydrogen.

**11.** Compounds according to Claim 1, characterized in that $R_4$ represents a hydrogen.

**12.** Compounds according to Claim 1, characterized in that $R_5$ and $R_6$ represent a hydrogen.

**13.** Compounds according to Claim 1, characterized in that $R_5$ is a linear or branched $C_1$ to $C_6$ alkyl radical.

**14.** Compounds according to Claim 1, characterized in that $R_5$ and $R_6$ represent a linear or branched $C_1$ to $C_6$ alkyl radical.

15. Compounds according to one of Claims 1 to 14, in the form of a salt which is acceptable in therapeutic use, characterized in that these salts are hydrochlorides, hydrobromides, sulphates, fumarates or maleates.

16. A compound chosen from:

1) 4-*o*-Tolyl-1-(tryptamine-5-O-carboxymethyl)piperazide hydrochloride
2) 4-(α,α,α-Trifluoro-m-tolyl)-l-(tryptamine-5-O-carboxymethyl)piperazide hydrochloride
3) 4-(Naphth-1-yl)-1-(tryptamine-5-O-carboxymethyl)-piperazide hydrochloride
4) 4-(2,3-Xylyl)-1-(tryptamine-5-O-carboxymethyl)-piperazide hydrochloride
5) 4-(m-Methoxyphenyl)-1-(tryptamine-5-O-carboxymethyl)piperazide hydrochloride
6) 4-Methyl-1-(tryptamine-5-O-carboxymethyl)piperazide hydrochloride
7) 1-[(tert-Butoxycarbamate)tryptamine-5-O-carboxymethyl]-4-[10-(8-methylthio-10,11-dihydrodibenzo-[b, f] thiepine)] piperazide hydrochloride
8) 4-*o*-Tolyl-1-[tryptamine-5-O-(α-phenylcarboxymethyl)]piperazide hydrochloride
9) 4-Naphthyl-1-[tryptamine-5-O-(α-phenylcarboxymethyl)]piperazide hydrochloride
10) 4-o-Tolyl-1-[tryptamine-5-O-(α-methylcarboxymethyl)]piperazide hydrochloride
11) 4-(Naphth-1-yl)-1-(tryptamine-5-O-methylsulphonyl)-piperazide hydrochloride
12) 4-*m*-Chlorophenyl-1-(tryptamine-5-O-propyl)piperazine hydrochloride
13) 4-(4-Acetylphenyl)-1-(tryptamine-5-O-carboxymethyl)-piperazide hydrochloride
14) 4-(2,4-Dimethylphenyl)-1-(tryptamine-5-O-carboxymethyl)piperazide hydrochloride
15) 4-(4-Chlorophenyl)-1-(tryptamine-5-O-carboxymethyl)-piperazide hydrochloride
16) 4-(3,4-Dichlorophenyl)-1-(tryptamine-5-O-carboxymethyl)piperazide hydrochloride
17) 4-(4-Methoxyphenyl)-1-(tryptamine-5-O-carboxymethyl)piperazide hydrochloride
18) 4-Cyclopentyl-1-(tryptamine-5-O-carboxymethyl)-piperazide hydrochloride
19) 4-(o-Tolyl)-1-(tryptamine-5-O-ethyl)piperazine hydrochloride
20) 4-(Naphth-l-yl)-1-(tryptamine-5-O-ethyl)piperazine hydrochloride
21) 4-(3,4-Dichlorophenyl)-1-(tryptamine-5-O-ethyl)piperazine hydrochloride.

17. Process for the preparation of the compounds of general formula (I) according to Claim 1 or of their salts or solvates which are acceptable in therapeutic use, characterized in that a compound of general formula (II):

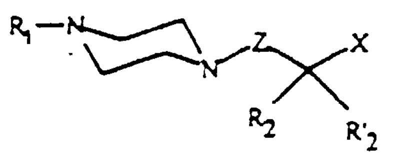

(II)

in which $R_1$, Z, $R_2$ and $R'_2$ have the meanings indicated with reference to the general formula (I) and where X is defined as a leaving group such as a halogen (bromine, iodine or chlorine), a mesylate, a tosylate or a triflate, is reacted with a serotonin derivative of general formula (III):

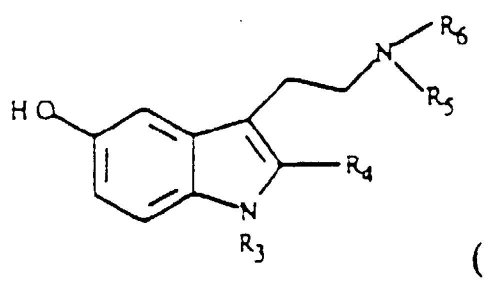

(III)

in which $R_3$, $R_4$, $R_5$ and $R_6$ have the meaning indicated with reference to the general formula (I) and $R_6$ is other than a hydrogen atom.

**18.** Process for the preparation of the compounds of formula (I) according to Claim 1 in which Z represents CO, characterized in that a compound of general formula (V):

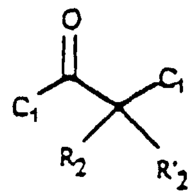

(V)

in which $R_2$ and $R'_2$ have the meaning indicated with reference to the general formula (I), is reacted with, first of all, a derivative of general formula (IV) :

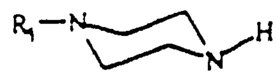

(IV)

in which $R_1$ is defined as in the general formula (I), and then with a derivative of general formula (III) according to Claim 17 in which $R_3$, $R_4$, $R_5$ and $R_6$ are defined as in the general formula (I).

**19.** Process according to either of Claims 17 and 18, characterized in that a compound of general formula (I) or a salt or protected derivative of such a compound is converted to another compound of general formula (I) and/or a compound of general formula (I) or a salt of such a compound is converted to a salt or solvate which is acceptable in pharmaceutical use.

**20.** Process for the preparation of the compounds of general formula (I) according to Claim 1, characterized in that a derivative of formula (IV) according to Claim 18 in which $R_1$ has the meaning indicated with reference to the formula (I) is reacted with a derivative of formula (VII):

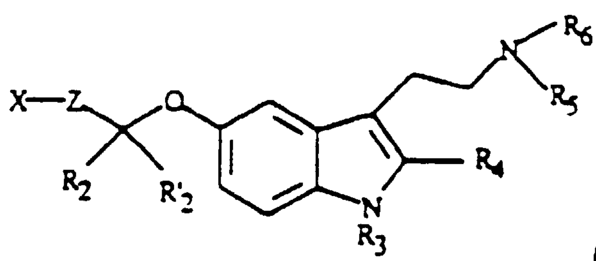

(VII)

in which Z, $R_2$, $R'_2$, $R_3$, $R_4$ and $R_6$ have the meaning indicated with reference to the formula (I) and $R_6$ is other than a hydrogen atom and in which X represents a leaving group such as a halogen, a mesylate, a tosylate or a triflate or the group X-Z represents an activated ester which is favourable for the formation of an amide.

**21.** Pharmaceutical compositions containing, as active ingredients, a compound according to one of Claims 1 to 16 in combination with an acceptable pharmaceutical vehicle for both the curative and preventive treatment of disorders related to serotonin.

**22.** Pharmaceutical compositions containing, as active ingredients, a compound according to one of Claims 1 to 16 in combination with an acceptable pharmaceutical vehicle for the treatment of depression.

**23.** Pharmaceutical compositions containing, as active ingredients, a compound according to one of Claims 1 to 16 in combination with an acceptable pharmaceutical vehicle for the treatment of compulsive obsessional disorders.

**24.** Pharmaceutical compositions containing, as active ingredients, a compound according to one of Claims 1 to 16 in combination with an acceptable pharmaceutical vehicle for the treatment of anxiety.

**25.** Pharmaceutical compositions containing, as active ingredients, a compound according to one of Claims 1 to 16 in combination with an acceptable pharmaceutical vehicle for the treatment of aggressiveness and/or alcoholism and/or asocial behaviour.

**26.** Pharmaceutical compositions containing, as active ingredients, a compound according to one of Claims 1 to 16 in combination with an acceptable pharmaceutical vehicle for the treatment of migraine.

**27.** Pharmaceutical compositions containing, as active ingredients, a compound according to one of Claims 1 to 16 in combination with an acceptable pharmaceutical vehicle for the treatment of eating disorders, in particular bulimia.

**28.** Pharmaceutical compositions, characterized in that they contain, as active substances, at least one compound according to any one of Claims 1 to 16 in combination with an acceptable pharmaceutical vehicle for the treatment of sexual dysfunctionings.